# EUROPÄISCHE PATENTSCHRIFT

(11) **EP 2 898 901 B1**
(45) Veröffentlichungstag und Bekanntmachung des Hinweises auf die Patenterteilung: **02.12.2020**
(21) Anmeldenummer: 14002544.6
(22) Anmeldetag: 23.07.2014
(51) Int. Cl.: A61L 2/025

(54) **REINIGUNGSVORRICHTUNG UND REINIGUNGSVERFAHREN**
Cleaning device and method
Dispositif de nettoyage et procédé de nettoyage

(30) Priorität: 19.11.2013 DE 102013019382
(43) Veröffentlichungstag der Anmeldung: 29.07.2015
(73) Patentinhaber: Simmoteit, Robert, 72414 Rangendingen (DE)
(72) Erfinder: Simmoteit, Robert, 72414 Rangendingen (DE)

(56) Entgegenhaltungen:
- EP-A1- 1 925 321
- EP-A1- 1 938 743
- EP-A1- 2 105 147
- EP-A2- 1 946 692
- US-A- 5 505 218
- US-A1- 2009 062 610

## Beschreibung

### Gebiet der Erfindung

Die Erfindung betrifft eine Reinigungsvorrichtung und ein Reinigungsverfahren zur Hohlrauminstrumentenaufbereitung, insbesondere für die medizinische Produktreinigung. Dabei findet die Erfindung überall dort Anwendung, wo Hohlrauminstrumente und Produkte mit Kanälen mit Reinigungsgeräten gereinigt werden.

### Stand der Technik

Reinigungsvorrichtungen können Spülmodule enthalten, die mobil oder fest im oder am Siebkorb angebracht sind. Ziel ist es, mit Zusatzkomponenten eine einfache Hohlrauminstrumentenreinigung im Siebkorb ohne Umpacken zu erreichen. Zu diesem neueren Stand der Technik gehören separate Spülleisten, die in Siebkörben eingesetzt werden. In DE 10 2004 029 970 und DE 10 2004 060 289 sind Reinigungsvorrichtungen beschrieben, die ein endständig funktionales Teil aufweisen, um die Vorrichtung mit einer Tragplatte oder ähnlichem zu verbinden. Als weiterführende Komponenten zur Durchspülung von Hohlrauminstrumenten werden in DE 10 2009 052 843 A1 Halterungsblöcke eingesetzt, um hiermit Anschlussadapter für Hohlrauminstrumente in der optimalen Orientierung anzuordnen. Ähnliche Reinigungsvorrichtungen werden auch bei der Endoskopaufbereitung in Reinigungsmaschinen eingesetzt. Andere Vorrichtungen so wie in US 5 476 454 beschrieben ermöglichen eine Medienweiterleitung durch ein Adapteranschlusssystem, das über seitliche Fixierungen festgelegt ist. In einer Spülmaschine sind Vorrichtungen bekannt, so wie in DE 10 2007 003 894 A1 gezeigt, die eine Kopplungsvorrichtung mit einem Anschlussstück aufweisen, dass relativ zu einem Befestigungsbereich am Spülgutträger beweglich angeordnet ist Weitere bekannte Reinigungsvorrichtungen sind in EP 1 925 321 A1, EP 1 946 692 A2, US 5 505 218, EP 2 105 147 A1 und EP 1 938 743 beschrieben.

Die obige Technologie hat jedoch den Nachteil, dass die mobilen Anschlusskomponenten im Siebkorb Platz benötigen und damit den Lagerungsplatz für medizinische Instrumente erheblich beeinträchtigen. Weiter haben die Nutzer dieser Technologie die Schwierigkeit, geeignete und flexibel nutzbare Adapter für den Instrumentenanschluss vorzufinden oder diese korrekt einzusetzen. Damit sind Fehler bei der Hohlraumaufbereitung die Regel, die Instrumentenreinigungsleistung ist beeinträchtigt und eine korrekte Validierung der Prozesse ist erschwert. Darüber hinaus sind die bekannten Systeme in der Regel eine auf die Anwendung abgestimmten Maschinentechnologie beschränkt, was die wirtschaftliche Nutzunmöglichkeit begrenzt.

### Beschreibung

Die Neuerung löst die bekannten Nachteile durch eine neuartige Reinigungsvorrichtung und mit einem neuartigen Reinigungsverfahren. Als eigenständige Erneuerung kommen vorgefertigte oder zumindest steckbare Reinigungsmodule, die individuell änderbar sind, zum Einsatz. Hierbei wird der Set-Gedanke mit eingezogen. Ein Set bedeutet, dass ein Siebkorb ein chirurgisches Set an medizinischen Instrumenten für einen medizinischen Eingriff enthält. Mit der Neuerung können die unterschiedlichsten Hohlrauminstrumente effektiv und wirtschaftlich gereinigt werden, ohne dabei die Mediumverteiler (Einsatzwagen, Spülleisten, Spülzylinder etc.) durch Zusatzadapter immer wieder anpassen zu müssen. Mit der Lösung wird weiter das Instrumenten- und Reinigungsmanagement optimiert, die Kosten für den Einsatz der Technologie reduziert und flexibilisiert sowie Fehler bei der Aufbereitung (Transport, Reinigung, Lagerung und Sterilisierung) vermieden. Die Neuerung verbessert darüber hinaus die Validierbarkeit der Instrumentenaufbereitung (Reinigen, Verpacken, Sterilisieren und Transportieren) durch Standardisierung bis hin zur Anwendung im OP.

Die Aufgabe wird erfindungsgemäß durch eine Reinigungsvorrichtung, nach Anspruch 1 und durch ein Reinigungsverfahren nach Anspruch 13 gelöst. Die abhängigen Ansprüche betreffen vorteilhafte Ausgestaltungen der Erfindung.

Die Erfindung löst die Aufgabe durch eine Reinigungsvorrichtung zur Hohlrauminstrumentreinigung, für die medizinische Produktreinigung und für den OP, aufweisen einen Siebkorb, mit Durchbrüchen und Korbadapter und einem Mediumverteiler mit Mediumverteileradapter und Mediumraum wobei die Korbadapter und die Mediumverteiladapter aufeinander abgestimmt sind, dadurch gekennzeichnet, dass zumindest die Reinigungsvorrichtung ein Fixierungsteil aufweist, wobei das Fixierungsteil eine von Hand drehbare Haltewelle, -stift, -stange oder - schraube ist und diese außen an einer Mediumwandung des Mediumverteilers geführt oder durch die Wandung des Mediumraums des Mediumverteilers geführt und gehalten wird damit das Fixierungsteil über eine von Hand steuerbare Anpresskraft den Mediumverteiler mit einer Siebkorbwandung, einem Siebkorbboden oder an einer dieser befestigten Haltefläche zumindest festlegbar ist und dabei eine Kopplung koppelbarer Korbadapter mit Mediumverteileradapter so erfolgt, dass dabei Adapterkanäle miteinander gekoppelt sind. Eine Festlegung und/oder Kopplung erfolgt dabei vorzugsweise durch Drehen und/oder Drücken oder eine Kombination aus diesen und kann über eine Feder, ein elastisches Element oder ähnliches unterstützt sein.

Die Neuerung sieht Reinigungsvorrichtungen vor, die auch dadurch gekennzeichnet sind, dass zumindest die Durchbrüche oder das Durchbruchsgitter der Vorrichtung eine definierte Adapter- und Halterungsanordnung und deren Ausrichtung vorgibt oder mitbestimmt oder in seiner Relation zum zu reinigenden Kanaldurchmesser eine Durchbruchsgröße zur Verfügung stellt, damit eine sichere Kanalreinigung und/oder gesetzlich geforderte Reinigung erfolgt kann. Falls hiervon abweichende Durchbruchsgitter oder vergrößerte Durchbrüche in der Vorrichtung vorhanden sind, sind diese mit Halteflächen so zu überbrücken, dass hieran Adapter, Halteadapter oder Fixierungsteile festlegbar sind. Darüber hinaus können über geeignete Durchbrüche in der Vorrichtung Adapter eines Mediumverteiler oder eines Mediumstecker von außen über Durchbrüche in die Vorrichtung eingebracht werden.

Die Gitter und/oder die Durchbruchsgrößen der Siebkörbe sind so zu wählen, das medizinische Instrumente sicher gelagert, gereinigt, spitze Instrumententeile nicht zu leicht durch das Gitter stechen und hierrüber wiederum genügend Reinigungsflüssigkeit geführt werden kann. Dabei ist die benötigte Reinigungsmenge z. B. über Reinigungsversuche zu ermitteln und ist abhängig von medizinischen Instrumentendesign und den innen verlaufenden Kanälen. Beispielsweise kann ein großlumiges Instrument mit einem inneren Kanal von größer 10 mm nicht ohne weitere Hilfsmittel über ein kleines Adapterlumen unter 3mm, insbesondere nicht im Liegen, sicher gereinigt werden. Optimale Gitter besitzen Durchbrüche zwischen 4 - 6 mm und bei größeren Durchbrüchen empfiehlt es sich diese mit einer Hilfskonstruktion, z. B. mit Metallstreifen zu überbrücken. Je nach Schaftlänge können eingebrachte Instrumente schräg oder sogar senkrecht gelagert oder zumindest durchspült werden. Eine Schrägstellung der gesamten Vorrichtung wird möglich, wenn die festlegbaren Mediumverteiler oder Mediumstecker von unten am Vorrichtungsboden angebracht sind oder geeignete Maschinenwagen mit schrägen Ebenen zum Einsatz kommen.

Das Zusammenspiel der Adapteranordnung in einem Siebkorb und die Adapteranordnung eines Medienverteilers erfordert eine hohe qualitative und präzise Abstimmung der steckbaren oder dichtenden Adapterteile. Die Abstände der Adapter zueinander müssen genauestens stimmen, damit z. B. eine Rändelschraube eines Mediumverteilers eine Kopplung herstellt und sich dabei nicht verkantet. Die Kopplung wird über das Drehen des Fixierungsteils (Rändelschraube) durch eine von Hand steuerbare Anpresskraft unterstützt. Darum sollten nur genaue abweichen. Es ist vorgesehen, Steckverbindungen in den steckbaren Adaptern über eine zusätzliche Anpressdichtung zu dichten.

Das Zusammenführen der aufeinander abgestimmten Adapteranordnung zu einer funktionierenden Reinigungsvorrichtung und genormten Anschlüssen verbessert einen wirtschaftlichen, sicheren und validierbaren Aufbereitungsprozess dadurch, dass diese im OP bzw. außerhalb einer Maschine einsetzbar oder herstellbar ist. Darüber hinaus wird das falsche Anschließen zumindest von Hohlrumprodukten verhindern oder zumindest reduziert. Letzteres reduziert und vereinfacht ebenfalls die benötigten Anschlussanweisungen für das Reinigungspersonal und minimiert das Verletzungsrisiko durch kontaminierte Instrumente (Personenschutz).

Ein geeigneter Mediumverteiler oder Mediumstecker besitzt einen runden oder eckigen Mediumraum mit einem vorderen oder seitlichen Mediumzugang und verteilt z. B. Flüssigkeit, Dampf oder Luft auf die ausführenden Adapter. Ferner können wässrige Lösungen, Trocknungsluft, Desinfektionsmedium etc. hierüber verteilt werden. Der Mediumraum nimmt somit Medium über einen Zulauf auf, mischt und verteilt diese auf die Adapter.

In Weiterbildung ist vorgesehen, das die Reinigungsvorrichtung dadurch gekennzeichnet ist, dass zumindest ein von Hand bewegbarer und eigenständig nutzbarer Mediumverteiler über eine Schlauchleitung (biegbare Leitung) mit Medium versorgt wird und eine Haltestange, Haltewelle oder ähnliches Fixierungsteil aufweist, das durch einen runden oder eckigen Mediumraum und dessen Wandungen (bevorzugt zwei Wandungen) oder seitlich geführt ist. Dabei übt das Fixierungsteil, z. B. mit einem vorderen Gewinde oder einen Ausformungsansatz eine von Hand steuerbare Anpresskraft aus, die auf das Halteteil oder eine Haltefläche durch drehen übertragen wird. Alternativ kann der Mediumverteiler auch eine vordere Haltenute aufweisen die in einem Vorrichtungsdurchbruch (Wand, Boden Haltefläche) greift, um diesen festzulegen.

Vorgeschlagen wird ein Fixierungsteil in Ausgestaltung, z. B. einer Rändelschraube mit oder ohne Dichtelemente und/oder eine Haltenute für Springringe. Diese kann auch Blöcke geführt sein. Alternativ können Clips, Federn, Blöcke, Steckadapter und/oder Dichtadapter die Kopplung oder Festlegung an einem Siebkorb oder ähnlichem unterstützen. Von Vorteil ist dabei, dass die Kopplung von Hand trennbar, koppelbar und die eingesetzte Anpresskraft von Hand steuerbar ist. Bevorzugt wird eine längere Schraube (Rändelschraube), Haltestange oder Haltestift eingesetzt. Diese wird durch den Mediumkanal oder -raum der Vorrichtung geführt und tritt hinten wieder aus. Damit wird eine platzsparende, einfach zu handhabbare und lösbare Verschraubung mit einer Siebkorbwand herstellbar ist. Vorteilhafterweise besitzt die Haltestange Dichtelemente, damit zumindest ein Mediumaustritt (flüssig und/oder gasförmig) unterbunden wird. Halteteile für drehbare Fixierungsteile können schraubbare Gewindeadapter, ein Haltefläche in Ausformung eines Streifen mit einem Gewinde oder ähnliches sein. Denkbar sind hier auch Schienen, Streifen oder Haltewinkel die über beabstande Lager eine drehbare Haltefläche bereitstellen. Die Haltefläche kann über die Vorrichtungsadapter und/oder über zusätzliche Verschraubungen mit einer Siebkorbwand oder einen Siebkorbboden verbunden sein. Alternativ kann auch nur ein geeigneter Durchbruch das Festlegen einer drehbaren Haltestange mit einem vorderen Formteil ermöglichen. Dabei wird die Haltestange mit Unterstützung einer Feder oder eines elastisches Anpresselement und dessen Rückstellkraft in einem Wanddurchbruch oder Bodendurchbruch z. B. eines Siebkorbs durch Drehen und/oder Drücken der Haltestange lösbar verbunden.

Es ist z.B. vorgesehen, dass die in einem Siebkorb oder Siebkorbbehälter enthaltenden Produktadapter oder Vorrichtungsadapter direkt mit einer vorderen abdichtbaren Mutter oder einer Halteplatte mit einer Siebkorbwand oder mit einem Siebkorbboden fest verschraubt oder über den Boden drehbar angeordnet oder individuell anordenbar sind. Hierbei kommen insbesondere mehrteilige Adapter oder einteilige einschraubbare Adapter zum Einsatz, die vorderseitig einen geraden oder konischen Kanal und/oder eine kaum hervorstehende abdichtbare Kanalöffnung aufweisen und rückseitig als Anschlussteil einen Schlauchadapter, Luer Lock weiblich oder Luer Lock männlich Adapter, Steckadapter, Kappenadapter, Verteilerlanzen und -düsen, Verschlussadapter oder Hülsenadapter und/oder Fixierungsadapter besitzen. Die vorgesehene abdichtbare Mutter ist vorne plan mit oder ohne eine umlaufende Dichtnute oder besitzt eine leichte Vertiefung. Damit derartige Vorrichtungen mit einer anderen Reinigungsvorrichtung festlegbar ist, kann der Siebkorb eine innenliegende von Hand drehbaren Haltestange oder eine Halteschraube, die über eine Siebkorbwandung oder einen Boden geführt ist, z. B. zur Festlegung an einer Mediumwand einer Maschine oder zur Festlegung eines Mediumverteilers besitzen.

Geeignete Vorrichtungsadapter sind dadurch gekennzeichnet, dass diese zumindest zur Festlegung ein Gewinde und eine Nute für einen Sicherungsring mit vorzugsweise einem elastischen Anpresselement aufweisen. Eine Rückstellkraft eines elastischen Anpresselements oder einer Anpressscheibe kann dabei die Handhabung unterstützen. Vorrichtungsadapter und Halteteil können ein Gewinde mit einer durchbruchsbezogenen Ausformung nach dem Gewinde aufweisen, um das Verdrehen des Halteteils oder der Adapter in der Vorrichtung zu unterbinden.

In Weiterbildung der Neuerung sind Spülgutträger in Ausgestaltung von Siebkörben, Spülkörben, Siebkorbbehältern oder Gestellen oder als eine Geräteebene oder ähnlichem oder eine Kombination aus diesen vorgesehen. Die Neuerung benötigt neuartige oder auf die Vorrichtungsdurchbrüche abgestimmt Adapter und Hilfskonstruktionen und wertet die Nutzungsmöglichkeiten von Siebkörben auf. Damit erhalten die so ausgestalteten Siebkörbe, integrierte platzsparende und flexible Behandlungseinrichtungen.

Geeignete Siebkörbe besitzen für die Befestigung von Adaptern geeignete runde, quadratische, ovale, rechteckige, sechseckige oder ähnliche Durchbrüche oder Gitter. Dabei können kreuzförmige oder vergrößerte Durchbrüche zum Einsatz kommen. Von Vorteil ist es, wenn individuell die Adapter angeordnet werden können. Nicht besetzte Adapter im Siebkorb werden mit Verschlusskappen oder über Einsteckteile verschlossen oder werden durch einen steckbaren Dichtadapter ersetzt. Im Siebkorbboden oder einer Siebkorbwand können ferner Öffnungen vorhanden sein, über die die Mediumverteiler über eine Art drehbarer Riegel oder vordere und vorstehende Haltenute aufweisen, damit hiermit der Mediumverteiler durch Einschieben festlegbar ist. Die technischen Verbindungsmöglichkeiten sind so zu wählen, dass das Einführen von Steckadaptern oder die Überdeckung von Adaptern leicht wieder zu lösen sind.

Die Neuerung sieht beispielsweise auch eine Reinigungsvorrichtung vor, die zumindest aus einem Spülgutträger (Siebkorb, Siebkorbbehälter, Spülgestell, Spülgeräteebene etc.) in Verbindung mit einem z. B. steckbaren Medienverteiler aufweist und dabei Medium über zumindest eine biegbare oder feststehende Zuführleitung herangeführt wird. Weiter können geteilte Adapteranordnung und zwar, jede für sich, über Zuleitungen für die Hohlraumproduktreinigung eingesetzt werden. Durch die eigenständigen Nutzungsmöglichkeiten, der Modularität und der Standardisierung der Adapter und der Spülmodule und/oder, Adapteranordnungsleisten) werden Kosten gespart und die Flexibilität und Nutzbarkeit der eingesetzten Systeme erhöht.

Die Erfindung ermöglicht insbesondere durch die Ausgestaltung und den Aufbau der Adapteranordnung in Spülkörben in Verbindung mit einem passenden und insbesondere eine von Hand koppelbaren Mediumverteilers (Spülmodule wie Spülleisten, Spülzylinder, Spülblöcke etc.), dass diese damit die Aufbereitung, insbesondere für schwierig zu reinigende Produkte verbessert, den Reinigungsprozess qualifiziert und organisiert und das Reinigungsmanagement optimiert. Einhergehend wird auch die Organisation durch Vorplanung der Anschlussteile für die Reinigung von Spülprodukten erstmals verbessert und es wird garantiert, dass die Aufbereitungserfordernissen bzw. die Herstellerangaben zur Produktreinigung eingehalten werden können.

Eine Adapteranordnung ist dabei definiert als eine festgelegte Anordnung von gleichen oder unterschiedlichen Adaptern und kann im engeren Sinne als Adapter-Magazin oder Adapterregister bezeichnet werden. Die Adapterkanäle von medizinischen Hohlraumprodukten werden hieran angeschlossen und/oder gehalten und hierüber gereinigt. Die eingesetzten Adapter des Registers besitzen einen inneren durchgängigen Kanal. Weiter können Verteiler- und Sprühdüsen eingesetzt sein, um hierüber gezielt Reinigungslösung auf Spülprodukte zu sprühen und/oder in Produktkanäle zu leiten.

Durch Koppeln zweier unterschiedlicher Adapter-Systeme beispielweise die von Siebkorbadapter für das Halten und/oder durchspülen von Hohlrauminstrumenten und die Adapter eines Mediumverteilers für das Einkoppeln in andere Adapteröffnungen, wird sichergestellt, dass keine Anschlussprobleme, beispielsweise in einem Reinigungsgerät (Reinigungsgerät, Ultraschallgerät) beim Reinigen und Trockenen, existieren. Dabei kann weiter vorgesehen sein, dass über die Adapteranordnung die hier eingebrachten oder angeschlossenen Spülprodukte gesichert und organisiert und nach dem Reinigen, für den Weitertransport gehalten werden und anschließend zusammen sterilisierbar sind. Erst im OP werden die sterilisierten Spülprodukte vom Adapter genommen und können nach der Operation hier auch wieder eingebracht werden. Geeignete Adapter können somit sterlisierbar sein und die Sterilisierung (z. B. die Hohlraumplasma-Sterilisierung) unterstützen.

Die Anordnung der Mediumverteiler-Adapter kann in der Fläche ein geometrisches Muster (Quadrat, Rechteck, Sechseck etc.), einreihig oder mehrreihig oder mehrerer Register bilden und in Beziehung zum Durchbruchsraster eines Siebkorbs angeordnet sein. Die Adapter und/oder Verteilerdüsen sind vorzugsweise mit einer Wand, einem Boden, einer Leiste oder ähnlichem lösbar festgelegt. Die Adapteranordnung kann die Entnahme und das Anschließen der medizinischen Instrumente in einem Siebkorb unterstützen. Ziel ist das Reinigen ohne Umpacken.

In Weiterbildung ist denkbar, dass die Adapteranordnung eine Organisationsstruktur für die medizinische Instrumentenaufbereitung unterstützt, unabhängig davon, ob Hohlrauminstrumente oder Instrumente ohne einen Kanal aufbereitet werden müssen. Die Spülgutträger erhalten hiermit eine sichtbare Prozess- und Lagerungsstruktur, die damit auch die OP-Führung beeinflusst oder auf diese abgestimmt ist. Damit ist gewährleistet, dass auch der Transport und die Sterilisierung sicher werden. Spülprodukte die nur eine Außenreinigung erhalten, können in der Orientierung zu einer Sprühdüse gelagert werden. Entsprechend können an den Adaptern in einem Siebkorb auch schwer zu reinigende Instrumententeile wie Bohrer etc. angeordnet sein. Ferner ist denkbar die Adapter oder die Halteflächen eine Nutzungskennzeichnungen aufweisen, damit die Einsortierung und/oder der Produktanschluss unterstützt sind.

Mediumverteiler können der Einfachhalber als Medienstecker (bzw. Spülmedienleistenstecker, Spülmedienzylinderstecker etc.) bezeichnet werden. Diese sind definiert als reine steckbare Medienverteiler (ähnlich einem Stromstecker), die zumindest zwei steckbare Adapter aufweisen. Die Neuerung bezieht Mediumverteiler (Spülmedien-Verteildichter z. B. eine Medienleiste oder Medienzylinder mit Dichtelementen) mit ein, die selbstdichtende vordere Anschlussteile besitzen, um nach vorne eine sichere Abdichtung zu erreichen. Die dichtenden Adapter (Anschlussteile) bzw. Elemente bestehen aus dichtendem Kunststoff, wie Silikon etc. und ermöglichen eine dichtende Überdeckung einer Vorrichtungsadapteröffnung. Die Dichtadapter können so gestaltet sein, dass diese das Einschieben in eine längliche Siebkorbwandung durch eine Rückstellkraft unterstützen. Die Mediumverteiler werden dabei beispielsweise aus einem runden, quadratischen oder rechteckigen Rohr oder Zylinder und hier angebrachten Steck- und/oder Dichtadaptern hergestellt. Auf den Spülrohrstecker oder Spülzylinderstecker etc. können 2 - 10 oder sogar mehr Steckadapter und/oder Dichtadapter angeordnet sein. Dabei erfolgt die Hauptmedienzuführung in die Reinigungsvorrichtung über Schlauch-, Steck- und/oder Luer Lock oder ähnlichen Anschlussadaptern zumindest ein- und/oder beidseitig. Der Zylinder besteht dabei z. B. aus zwei Teilen und kann nach hinten oder auch seitlich eine Medienzuführung aufweisen. Je nach Bauweise kann der Zylinder auch einen Filter beinhalten.

Die Anzahl und die Ausrichtung der Medienzuleitungen am Mediumverteiler orientieren sich nach der benötigten Medienmenge. Dabei kann der Mediumstecker oder der Spülmedien-Verteildichter auch als eine mobile flächige Doppelwandung (Medienwand) ausgebildet sein und diese wiederum ein Bestandteil eines Einschubwagens einer Reinigungsmaschine sein oder in diese eingebracht werden. Die Medienwand selber kann wiederum über große Mediensteckadapter mit Reinigungsmedium versorgt werden. Um Unklarheiten zu vermeiden, wird vorzugsweise dann von einer Medienwand gesprochen, wenn diese zumindest eine Spülebene eines Einschubwagens ein- und/oder beidseitig zu mindestens 20 % einer Seitenfläche bildet und/oder in seine Querschnittsmaße die Wandhöhe mindestens viermal der Wandbreite entspricht.

Von Vorteil ist, wenn eine mobile Medienwand in einem Etagenwagen von unten, oben oder seitlich mit Reinigungsmedium versorgt wird. Eine derartige Konstruktion kann einen großen leistungsfähigen flächigen Partikel-Filter bei zu hoher Partikelbelastung der wässrigen Reinigungslösung enthalten. Leistungsfähige Partikelfilter können in einer Medienkammer einer Medienwand in einem Etagenwagen oder in einem Ultraschallbad angeordnet sein. Die Medienkammer kann dabei mehrteilig oder zumindest nach hinten geöffnet werden, damit der Filter leicht entnommen werden kann. Von Vorteil kann es sein, wenn die Medienwand leicht austauschbar ist und somit die Funktionalität von Reinigungsvorrichtungen, wie z. B. eines Etagenwagens, verbessert. Von Vorteil kann weiter sein, wenn verstärkt mit Verteilerdüsenregister (Sprühdüsen) Reinigungslösung ortsbezogen (am Ort, wo die eigentliche Reinigung erfolgt) verteilt wird. Dies wiederum reduziert die Baugröße von Reinigungsmaschinen erheblich und damit auch die Handhabbarkeit, weil derartige Filter leichter auszutauschen sind, als wenn diese im Inneren der Maschine angeordnet sind.

Rechteckige oder quadratische Medium- oder Mediumverteilerkammern (z. B. als ein mobiler Flach- oder Mediumstecker ausgebildet) können hinten oder seitlich einen oder mehrere Medienanschlüsse aufweisen und an der Vorderseite Steck- und/oder Abdichtadapter besitzen. Im Falle mehrerer Zuläufe ermöglicht dieser das Mischen von Reinigungsmedien (Luft mit Flüssigkeit) mit dem Ziel, die Reinigung effektiver zu gestalten.

Einfacher kann die Medienzuführung der Reinigungsvorrichtung über einen U-Adapter oder einen Doppelanschlussadapter erfolgen. Mit U-Adaptern können zwei Reinigungsmedien und/oder unterschiedliche Reinigungsmedien im Mediumverteiler vermischt werden, um das Innenlumen eines medizinischen Instrumentes zu reinigen. Vorteil dieser Medienzuführung ist z. B. eine bessere Verwirbelung des Reinigungsmediums schon vor dem Mediumraum.

Es ist denkbar, dass die geeigneten und vorzugsweise auch tragbaren Reinigungsvorrichtungen elektronische Sensoren zur Bestimmung der Leitfähigkeit, der Temperatur oder des Mediendruckes und/oder Ultraschallteile zur Bestimmung der Medienzusammensetzung enthalten. Diese weiterführende Ausgestaltung ermöglicht es wichtige Reinigungsparameter zu erfassen. Zusätzlich können RFID-Systeme zum Einsatz kommen die z.B. am Adapter oder an einem Halteteil angebracht sein können.

Die Erfindung löst die Aufgabe weiter durch ein Reinigungsverfahrengemäss Anspruch 13.

Die Weiterbildung des Reinigungsverfahrens, insbesondere für Reinigungsmaschinen, ist dadurch gekennzeichnet, dass zumindest über einen zweiteiligen Medienanschluss, U-förmigen Medienanschluss oder einen Doppelanschlussadapter Reinigungslösung und gepulste Luft oder Dampf in eine Reinigungsvorrichtung (Rohrverteiler oder Zylinder) eingebracht wird, um angeschlossene medizinische Instrumente zu reinigen.

Verfahrenstechnisch können Reinigungsvorrichtungen hergestellt werden, mit denen ohne Umpacken der enthaltenen Hohlraumprodukte diese in unterschiedlichen Reinigungsgeräten und Reinigungsverfahren gereinigt werden. Sinnvoll ist die Ultraschalreinigung und die Spülreinigung in Reinigungsautomaten miteinander zu verbinden und damit die Herstellung von Reinigungsstraßen zu ermöglicht. Der Vorteil von Reinigungsstraßen ist, dass diese effektiv und wirtschaftlich reinigen können. Multifunktional wird das Spülverfahren, wenn eine oder mehrere Medienausleitungen in unterschiedlichen Ebenen eines Etagenwagens oder in einem Ultraschallkorb in einem Ultraschallbad vorhanden sind und hierüber auch unterschiedliche Medien getrennt voneinander einleitbar sind. Da in einem Ultraschallbad und einer herkömmlichen Reinigungsmaschine die gleiche mobile Reinigungsvorrichtung einsetzbar ist, werden die Reinigungsverfahren miteinander sinnvoll verbunden und die Reinigungsleistung für Spülgüter und deren Validierbarkeit wesentlich verbessert.

Zusammenfassend lässt sich sagen, dass die Erfindung standardisierte, variable und mobile Reinigungsvorrichtungen herstellt und in Verbindung mit geeigneten Maschinenanschlüssen die medizinische Instrumentenaufbereitung und insbesondere die Standardisierung der benötigten Mediumverteiler verbessert.

### Einsatzgebiete und Ausführungsformen

Die Erfindung findet Anwendung bei der Reinigung von Hohlrauminstrumenten und insbesondere im Bereich der Augen-, HNO-, MIC- oder Bohrinstrumete oder Instrumententeile sowie bei komplexen Instrumenten, wie die der Endoskope oder der DaVinci-Instrumente.

Durch standardisierte Mediumverteiler und Mediumstecker in Verbindung mit z. B. Siebkorbreinigungsvorrichtungen und den hier angeordneter Adapter lassen sich die bekannten Reinigungsprozesse in der Medizintechnik besser auf die Kundenbedürfnisse anpassen. Darüber hinaus wird hiermit eine flexible Gestaltung oder die Verwendung von Einschubwagen in einer Reinigungsmaschine vereinfacht oder die Funktionsmöglichkeiten von Siebkörben erweitert.

Entsprechend können die hohen gesetzlichen Anforderungen bei der Reinigung von medizinischen Instrumenten besser eingehalten werden.

Anwendungsbeispiele und Ausgestaltungen geeigneter Mediumverteiler einer Reinigungsvorrichtung:
1. Runde, quadratische oder rechteckige Spülrohre oder Spülräume oder eine Medienwand eines Gerätewagens mit koppelbaren Adaptern.
2. Zylindrische mehrteilige Reinigungsvorrichtungen mit zumindest einem seitlichen oder unteren Medienanschluss als Mediumverteiler.
3. Verteiler, der über eine Schraube oder eine Haltestange oder einen Haltestift mit oder ohne Halterungsblock anschraubbar oder über zumindest eine vordere Haltenute in einem Durchbruch einschiebbar ist und gehalten wird.
4. Verteiler, der durch Drücken über eine Stange, einen Clip oder über Riegel o. ä. z. B. durch Drücken und/oder drehen festlegbar ist.
5. Verteiler, der Abdichtelemente aus Kunststoff besitzt.
6. Verteiler, der seitlich und/oder von unten mit einer Siebkorbwandung verbunden werden kann.
7. Verteiler, der zusätzlich einen Filter enthält und/oder Hähne oder Ventile und/oder eine elektronische Komponente aufweist.
8. Verteiler mit einer U-förmigen Medienzuleitung zum Anschluss für zwei gleiche oder ungleiche Hauptmedienzuführleitungen in dem Medienverteiler für die Vergrößerung der Medienmenge oder zur Vermischung von unterschiedlichen Medien.

Anwendungsbeispiele und Ausgestaltungen zumindest geeigneter Vorrichtungs- und/oder Mediumverteiler -Adapter:
1. Adapter hergestellt als einteilige Gewindeadapter oder als Schweißadapter und/oder zweiteilige oder mehrteilige Adapter (Adapter mit Mutter Adapter mit einer Wellensicherungsscheibe und/oder Dichtteile), die zumindest eine dichtende Verbindung mit einem Mediumverteiler ermöglichen.
2. Adapter mit einem Luer Lock (männlich oder weiblich Anschlussteil), Schlauch, Kappen- oder Steckadapters etc. als Instrumentenanschlussteil.
3. Adapter mit einer sich verjüngenden oder konischen Nute zumindest zur Herstellung einer Klemmverbindung mit einer Wandung.
4. Adapter mit einem Aufsatz- bzw. Anpresselement zur Stabilisierung einer lösbaren Adapterverbindung mit einer Siebkorbwandung.
5. Adapter an einer festlegbaren und/oder drehbaren Leiste oder einer Halteplatte die z. B., mit einem Siebkorb, Gestell oder einer Geräteebene fest verbunden oder lose eingelegt sind.
6. Adapter die geometrisch oder in Reihe an Halteflächen liegen und die hier Spülprodukte halten.
7. Adapter die an einer Siebkorbwand oder einem Siebkorbboden und/oder an separaten Leisten oder Halteplatten zu einem Adaptermagazin angeordnet sind.

Anwendungsbeispiele und Ausgestaltungen einer Reinigungsvorrichtung:
1. Reinigungsvorrichtung in Verbindung mit einem Adapter nach einem der Punkt 1 - 7 und einem Mediumverteiler nach einem der Punkte 1 - 8 in Verbindung mit zumindest einem Instrumentenkorb, -behälter oder -gestell, einer Geräteebene oder ähnlichem oder einer Kombination aus diesen.
2. Reinigungsvorrichtung, die eigenständig nutzbar sind (Mediumverteiler mit Adaptern und Siebkorb, Gestell oder Waschgutträger mit einer Adapteranordnung an einer Siebkorbwand und/oder einer Leiste).
3. Reinigungsvorrichtung, die die Reinigung von definierten OP-Sets mit Haltevorrichtungen für medizinische Instrumente, damit ohne Umpacken auch die Reinigung und Aufbereitung komplexer medizinischer Instrumente, wie flexible Endoskope, Phaco-Handstücke, Motorsysteme, medizinische Geräte etc., ermöglicht.
4. Reinigungsvorrichtung, die in einem Reinigungsgerät (Reinigungsautomat, Ultraschallgerät etc.) oder auch außerhalb dieser eingesetzt ist.
5. Reinigungsvorrichtung, die transportierbar und damit auch tragbar und beweglich ist.
6. Reinigungsvorrichtung, die sterilisierbar ist
7. Reinigungsvorrichtung mit Instrumentenkennzeichnung und/oder geeigneten Halterungen (Silikonnoppenbänder, Metallhalter etc.) zur besseren Rücksortierung der Spülprodukte, um damit den Aufbereitungs- und OP-Prozess unterstützt.
8. Reinigungsvorrichtung, über die eine Gerätesteuerung durch elektronische Komponenten und/oder über eine mechanische Kopplung erfolgen kann.
9. Reinigungsvorrichtung für die Entfernung von Restmedium nach dem Reinigen zumindest außerhalb einer Maschine oder zum Nachtrocknen oder zum Nachbehandeln (Instrumentenpflege etc.)
10. Reinigungsvorrichtung die von Hand festlegbar und tragbar und/oder ein Maschinenteil zumindest eines Einsatzwagens ist.
11. Reinigungsvorrichtung, die durch gezielte und ortsbezogene Mediumzuführung (Reinigungslösung) die Produktreinigung verbessert.
12. Reinigungsvorrichtung die ein Siebkorb oder eine Siebkorbbehälter oder ein Gestell ist und die Vorrichtungsadapter enthält und zumindest innenliegend eine Haltestange aufweist, die an eine Siebkorbwandung fixiert und/oder durch diese geführt ist.

Die obigen Ausgestaltungen der Komponenten bestimmen zumindest mit, wie das Reinigungsverfahren auszugestalten ist. In erster Linie ist ein Reinigungsverfahren, welches in Reinigungsgeräte zum Einsatz kommt, bevorzugt Die Einsatzgebiete des Verfahrens sind jedoch nicht nur auf die Reinigung und Aufbereitungsprozesse von medizinischen Produkte beschränkt.

Anwendungsbeispiele zur Ausgestaltung eines Reinigungsverfahrens:
1. Reinigungsverfahren, bei dem zumindest eine Reinigungsvorrichtung mit Adaptern einer Siebkorbwandung oder über eine Leiste festgelegt wird, so dass mit hohen Spül- und Dampfdrücken zwischen 0,5 und 5 Bar gearbeitet werden kann.
2. Reinigungsverfahren, bei dem zumindest ein Spülrohr mit einer geeigneten Siebkorbwand durch Eindrehen in diese Wandung festgelegt wird, um nach außen hin eine Anschlusskopplung für ein geeignetes Kopplungsteil in einem Einschubwagen für eine Reinigungsmaschine herzustellen.
3. Reinigungsverfahren, bei dem über Zuführleitungen, wie z. B. Schläuche o. ä., Medien in Instrumentenhalterungen oder deren Nähe (z. B. über Sprühverteiler) geleitet werden, damit zumindest die Außenreinigung der hier gelagerten Instrumente verbessert wird.
4. Reinigungsverfahren zur Produktdesinfektion, dadurch gekennzeichnet, dass über die Reinigungsvorrichtung Desinfektionsmedium geleitet werden kann, um zumindest eine Innenlumen-Sterilisation durchzuführen.
5. Herstellung einer Medienverbindung durch Anpressen oder Andrücken einer Reinigungsvorrichtung an eine Wandung mit Adaptern.
6. Reinigungsverfahrens, bei der zwei unterschiedliche Medien (wässrige und dampfförmige und/oder Luft) zur Reinigung in Instrumentenlumen eingesetzt werden.
7. Reinigungsverfahren, bei dem Filter zur Medienreinigung eingesetzt sind.
8. Reinigungsverfahren, bei dem zumindest die innenliegenden medizinischen Instrumente im Siebkorb über die enthaltenden Adapter angeschlossen sind und damit eine Lagerungsstabilisierung erreicht wird.
9. Reinigungsverfahren, bei dem zumindest Mediumstecker (Spülmedienleistenstecker oder Spülmedienzylinderstecker) und Siebkörbe mit Adaptern (Adapterkörbe) eingesetzt sind, um hierüber unterschiedliche Reinigungstechnologien, wie beispielsweise die Ultraschall- und Spülreinigungstechnologie zu verbinden.
10. Reinigungsverfahren, bei dem im letzten Schritt eine Nachbehandlung (Trocknung etc.) außerhalb einer Reinigungsmaschine erfolgt. Dabei kann weiter Pflegemedium innerhalb in den Mediumraum der Vorrichtung eingebracht werden.

Eine bevorzugte Ausgestaltung einer Reinigungsvorrichtung ist ein Siebkorb mit definierten Durchbruchsmuster oder Gitter, z. B. auch mit seitlichen biegbaren Funktionsteilen. In die wandständige Durchbrüche werden z. B. Luer Lock männliche, Kappen- und Schlauchadapter eingebracht. Der Abstand der Adapter ergibt sich durch das gleichmäßige Durchbruchsrater. Eine auf dieses Abstandsraster angepasste Anordnung von Steckadaptern bzw. Stecklanzen am Mediumverteiler (Spülmodul) erfolgt durch eine lösbare und mechanisch gestützte Kopplung. Diese erfolgt vorzugsweise mit einer durch das Spülrohrlumen bzw. durch einen Mediumraum gehende Rändelschraube, einem Haltestift oder einer Haltestange oder ähnlichem. Die Haltestange ist drehbar und mit Dichtelementen und einer Wellenscheibensicherung versehen, damit diese eine Einheit mit dem Spülrohr bildet. Das Spülmodul wird von Hand an eine Siebkorbwand festgelegt und über vorhandene Dichtungen am Steckadapter eine Abdichtung erreicht. Alternativ können hier die Steckverbindungen selbst oder andere drehbare Teile (Drehkappe etc.) an den Adaptern dafür sorgen, dass das Spülmodul an der Siebkorbwand gehalten wird. Um einen zu großen Druckabfall zu vermeiden, sind nicht genutzte wandständige Adapter verschlossen. Diese gesamte Vorrichtung wird in einer Reinigungsmaschine (RDG, Ultraschallbad etc.) z. B. über Schläuche oder direkt angeschlossen.

Bei einer weiteren Ausgestaltung einer Reinigungsvorrichtung wird ein Mediumverteiler eingesetzt, der über einen U-Mediumadapter mit Medium versorgt wird. Die Reinigungsvorrichtung wird links und rechts oder mittig z. B. über einen Block geführte Rändelschraube mit einer Siebkorbwand verbunden. In der Reinigungsmaschine wird an einem Arm des U-Adapters Reinigungsmedium und am anderen Dampf angeschlossen. Eine Festlegung ist hier wegen der hohen Drücke sinnvoll. Alternativ kann der Block eine vordere Nute aufweisen. Über die Blocknute erfolgt durch Einschieben in einen Siebkorbdurchbruch eine Festlegung des Mediumverteilers.

Eine andere Ausführung einer Reinigungsvorrichtung enthält eine Adapteranordnung, die mit dem Siebkorbboden oder einer Siebkorbseitenwand verschraubt ist. Die Registerleiste kann feststehend aber auch über seitliche Halterungen drehbar ausgestaltet oder nur eingelegt sein. Das Adaptermagazin kann ferner ein elektronisches RFID-System besitzen. Über RFID-Systeme kann angezeigt werden, wo sich die Vorrichtung im Aufbereitungsprozess befindet. Alternativ kann eine drehbare Registerleiste auch in der Mitte verjüngt sein, um hier über einen Block eine Drehbarkeit herzustellen. Die drehbare Ausführung einer Adapteransammlung dient vornehmlich zur besseren Orientierung der hier angeschlossenen Produkte oder dessen Halterung und sterilen Entnahme. Vorzugsweise sind hier die Gewindeadapter eingeschraubt.

Für die Gestaltung eines Reinigungsverfahrens wird ein zylindrischer Medienverteiler mit einem Filter und Steckadapter eingesetzt. Der Verteiler wird über die Steckadapter in die vorderen Öffnungen festgelegter Siebkorbadapter gesteckt. Im Siebkorb können lange Instrumente über Instrumentenhalterungen gesichert sein (z. B. lange Da Vinci-Instrumente, flexible Endoskope oder andere komplizierte Hohlraumprodukte). Nach dem Anschließen dieser Instrumente wird die Vorrichtung in einer Reinigungsmaschine eingebracht und die benötigten Reinigungsmedien an der Reinigungsvorrichtung über Schläuche angeschlossen. Nach der erfolgten Reinigung wird der Siebkorb als auch eigenständig nutzbare Reinigungsvorrichtung aus der Maschine genommen und mit Druckluft außerhalb der Maschine über den Medienzugang an der Reinigungsvorrichtung das Restwasser entfernt.

Die Erfindung kann bei der Instrumentenaufbereitung im Pharma- und Laborbereich sowie überall dort angewendet werden, wo zumindest Spülgüter mit Kanälen entsprechend ihrem Aufbau effektiv gereinigt werden müssen.

### Ausführungsbeispiele

Im Folgenden wird die Erfindung anhand von Ausführungsbeispielen im Zusammenhang mit der Zeichnung ausführlicher erläutert.
- Fig. 1: zeigt eine Reinigungsvorrichtung mit unterschiedlichen Vorrichtungsadapteranordnungen in einer Drauf- und einer Seitenansicht.
- Fig. 2: zeigt unterschiedliche Vorrichtungsadapter, die mit Durchbrüchen oder über Gewinde mit einer Wandung oder einer Haltefläche lösbar verbunden werden können.
- Fig. 3.: zeigt unterschiedliche Halteflächen und Teile zum Anbringen von Vorrichtungsadaptern.
- Fig. 4: zeigt zwei in Schnittdarstellung aufeinander abgestimmte Adapteranordnung.
- Fig. 5: zeigt in einer Draufsicht einer Siebkorbreinigungsvorrichtung mit zwei teilbaren Reinigungsvorrichtungen die über biegsame Zuleitungen mit Reinigungsflüssigkeit versorgt werden.
- Fig. 6: zeigt eine Reinigungsvorrichtung in Schnittdarstellung bestehend aus einem Siebkorb mit unterschiedlich gestalteten Medienwänden eines Gerätewagens.
- Fig. 7: zeigt eine Reinigungsvorrichtung mit einem Siebkorb und einem zylindrischen Mediumverteiler.
- Fig. 8: zeigt unterschiedliche mobile Rohrverteiler (Spülleisten bzw. Spülmodule).
- Fig. 9: zeigt unterschiedliche eckige Mediumstecker.
- Fig. 10: zeigt Siebgitter mit im Boden angeordneten Mediumverteilern und unterschiedliche Durchbruchsrastern.

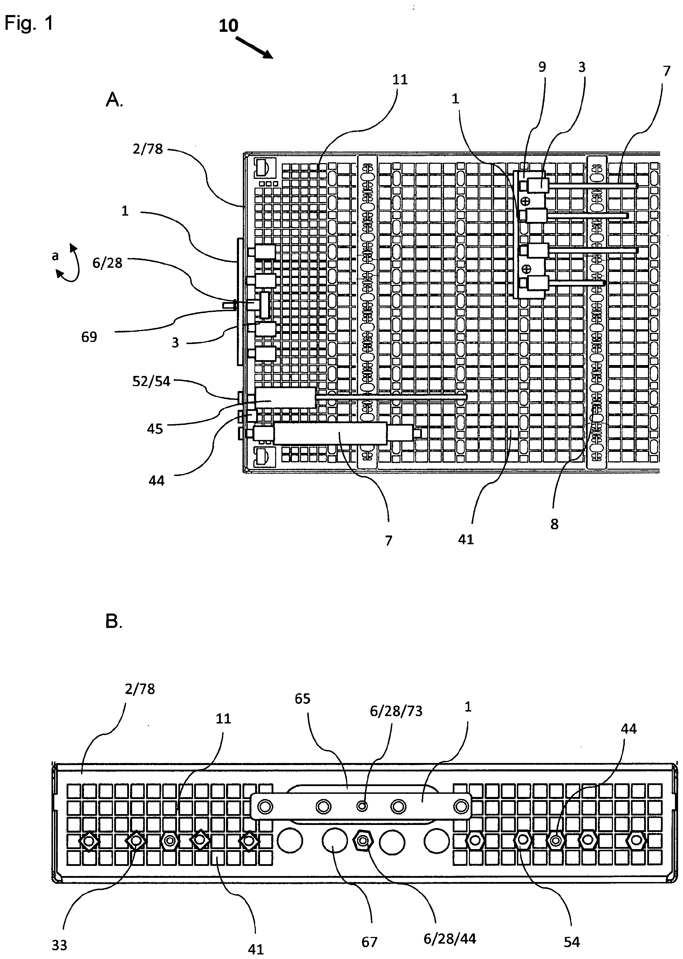

Figur 1 zeigt eine Reinigungsvorrichtung mit unterschiedlichen Vorrichtungsadapteranordnungen in einer Drauf- und einer Seitenansicht. Die Darstellung **A.** ist eine eigenständig nutzbare Reinigungsvorrichtung **10** zumindest, wenn diese mit zumindest einer Mediumzuleitung mit Reinigungsflüssigkeit versorgt wird. Über eine Haltefläche, in Ausgestaltung einer flächigen Halteplatte (Metallstreifen) **1** und einer abgewinkelten Adapterhaltefläche **1** mit zwei beabstandeten Halterungsteilen **9** sind unterschiedliche koppelbare Adapterteile **3** angeordnet. Die angebrachten Vorrichtungsadapter zeichnen sich dadurch aus, dass diese zum Teil über die Siebkorbwanddurchbrüche **2** mit einer Halteplatte **1** verschraubt sind und diese damit auch halten. Im Siebkorb der Vorrichtung **10** werden medizinische Produkte **7** wie z. B. Bohrer ohne Kanäle über Adapter (z. B. selbstverschließbare Kappenadapter **45**) zur besseren Spitzenreinigung gehalten und dabei über Lamellenhalterungsstreifen **8** oder alternativ über Noppenstreifen etc. positioniert und gelagert. Dabei ist vorgesehen, dass die Vorrichtungsadapter über flache Spezialmuttern **54** über Durchbrüche mit der Siebkorbwand **2** bzw. mit dieser Vorrichtungswand **78** direkt verschraubt sind. Der Fixierungsadapter **44** unterstützt die Fixierung eines mobilen Mediumverteilers oder kann ein Anschlussadapterteil festlegen. Die Vorrichtung **10** besitzt eine Fixierungsteil **6** (Rändelschraube etc.) mit einem verbreiterten Schaft **28** (Welle, Stift, Stange etc.) und ist mit einem Sicherungsring **69** drehbar und/oder drückbar fixiert. Mit diesem von Hand drehbaren Fixierungsteil **6** kann die Vorrichtung **10** mit einer Haltevorrichtung oder Halteteil in einer Mediumwand einer Maschine oder mit einem mobilen Mediumverteiler der z. B. ein Haltegewinde etc. aufweist, gekoppelt werden. Ein Mediumverteiler wird dabei von der Innenseite des Siebkorbes her über die Siebkorbwandung **2** gehend festgelegt. Die weiteren Halteteile **9** sind mit Schrauben mit dem Siebkorbboden über quadratische oder ovale Bodendurchbrüche **41** lösbar verbunden. Darstellung **B.** zeigt eine ähnliche Reinigungsvorrichtung mit außen an der Siebkorbwand **2** angelagerten Adapterhalteflächen, z. B. in Ausgestaltung von Flachmuttern **54,** quadratischen Ansatzelementen **33** und einem Halttestreifen **1.** Darüber hinaus sind in der Siebkorbwandung **2** oder **78** weitere größere Durchbrüche **65** und **67** gezeigt, die größer sind als die Siebkorbwandurchbrüche **11.** Auch der Handdurchbruch **65** kann mit einem Haltestreifen **1** für die Platzierung von Vorrichtungsadapter über eine Halteplatte genutzt werden. Über die Durchbrüche **67** können Mediumverteiler-Adapter direkt in das Siebkorbinnere eingebracht und über eine Fixierungsadapter **44** hier festgelegt werden. Durch Drehen eines vorderen Adapteransatzelementes **33** im Siebkorbdurchbruch **41** des Siebkorbgitters **11** können ebenfalls Vorrichtungsadapter mit der Siebkorbwandung lösbar verbunden werden. Die Reinigungsvorrichtung **10** besitzt weiter eine, durch den Haltestreifen **1** gehendes und nach hinten geführtes Fixierungsteil **6,** deren Gewinde im Schaftbereich **28** über ein Gewindeloch **73** herausgeführt wird. Alternativ kann auch ein Fixierungsadapter **44** diese Aufgabe übernehmen. Mehrgängige Gewinde können dabei die Handhabung verbessern.

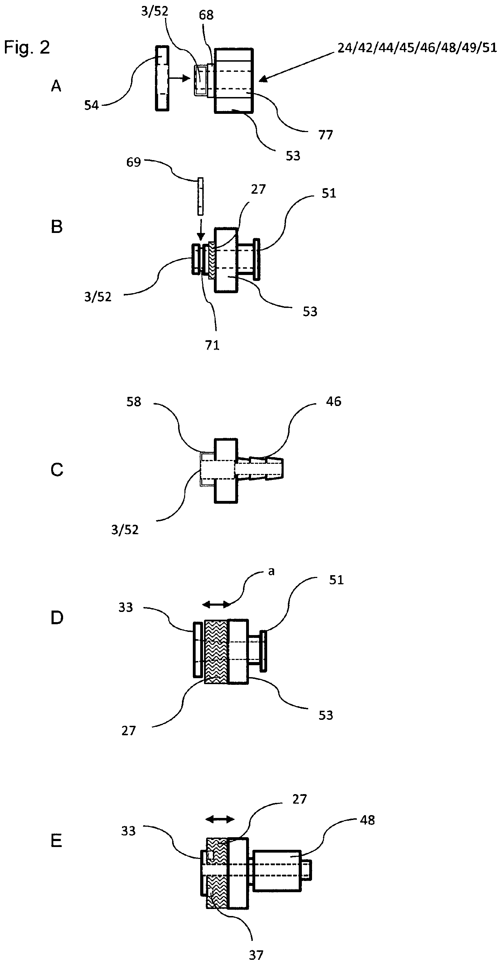

Figur 2 zeigt unterschiedliche Vorrichtungsadapter, die mit Durchbrüchen oder über Gewinde mit einer Wandung oder einer Haltefläche lösbar verbunden werden können. In **A** ist ein koppelbarer Vorrichtungsadapterteil **3** bzw. ein Fixierungsadapter **44** gezeigt, der aus zwei Teilen besteht. Der Adapterkörper **53** kann mit dem vorderen kurzen Außengewindeteil mit einer Mutter **54** verschraubt werden. Darüber hinaus kann der Fixierungsadapter **44** auch als Verbindungsadapter eingesetzt sein. Der Adapterkörper **53** kann dabei einen Schlüsselansatz **77** und kann ein durchgehendes Innengewinde und/oder einen Adapterkanal **52** aufweisen. Hier können Haltewellen, Haltestangen, Schrauben oder Gewindestifte zur Halterung einer Reinigungsvorrichtung über ein Innengewinde oder im Falle einer geeigneten Kanalöffnung, können hier Steckadapter eingebracht werden. Der hintere Adapterteil kann zumindest zum Anschluss von Hohlrauminstrumenten als Mediumansatz **24,** als Spülansatz für eine Spülhülse **42,** als eine Spüllanze **43,** mit einem durchgehenden Innengewinde als ein Fixierungsadapter **44,** als reiner Kappenadapter **45,** als Schlauchadapter **46,** als Luer Lock männlich Adapter **48,** als Verschlussadapter **49** oder als Luer Lock weiblichen Adapter **51** ausgebildet sein. Darüber hinaus besitzt der Adapterkörper **53** nach dem Außengewinde einen z. B. quadratischen Ansatz **68,** um diesen zusätzlich mit einem quadratischen Durchbruch einer Vorrichtungswandung festzulegen. Über die Schraubadapter ist eine individuelle Verschraubung der Vorrichtungsadapter mit Durchbrüchen einer Siebkorbwandung möglich. Entsprechende hintere Adapteranschlussausführungen sind mit den folgenden wandständigen Vorrichtungsadaptern **B, C, D** und **E** möglich. In **B** ist ein Adapter gezeigt, der vorne eine Wellennute zum Einbringen einer Sicherungsring **69** und zur Halteunterstützung einen elastischen Halteteil und/oder Dichtteil **27** (Silikonring, Kunststoffscheibe etc.) besitzt, um den Adapterkörper **53** besser mit Durchbrüchen in einer Vorrichtungswandung bzw. Vorrichtungsboden zu verankern. Abbildung **C.** zeigt einen einfachen Schlauchadapter **46** mit einem Außengewinde **58** und unterschiedlichen Kanaldurchmessern **52.** Abbildung **D.** zeigt einen über eine Rückstellkraft festlegbaren neuartigen Anschlussadapter (z.B. Luer Lock weiblich Adapter) der ein zusätzliches Halte, Dicht- und Anpresselement **27** besitzt. Über das elastische Element **27** und dessen Rückstellkraft wird das steckbare Adapterende in Ausgestaltung einer ovalen bzw. rechteckigen Platte **33** oder in z. B. einer quadratischen Ausgestaltung über einen Durchbruch in einer Vorrichtungswand (Lochblech eines Siebkorbs) lösbar verbunden. Diese Halteelement **33** kann aber auch als steckbarer Ansatz (z. B. als eine Metallkunststoffverklebung, Verbundmaterial) ausgebildet sein. Abbildung **E** zeigt einen anderen Adaptertyp mit einer Halterungsnute oder Haltestiftansatz **37** und eine vorderen z. B. ovale Halteplatte **33** der ebenfalls über ein elastisches Element **27** stabil und drehbar mit einer Siebkorbwand verbunden werden kann. Die Festlegung wird auch hier durch eine Rückstellkraft, die beim Eindrehen in eine Blechwandung erzeugt wird (Presspassung), wobei die vordere Nute **37** oder ein Ansatzteil ein verdrehen des Adapters verhindert. Denkbar sind für die Adapter **D** und **E** auch Federn, anstelle elastischer Elemente.

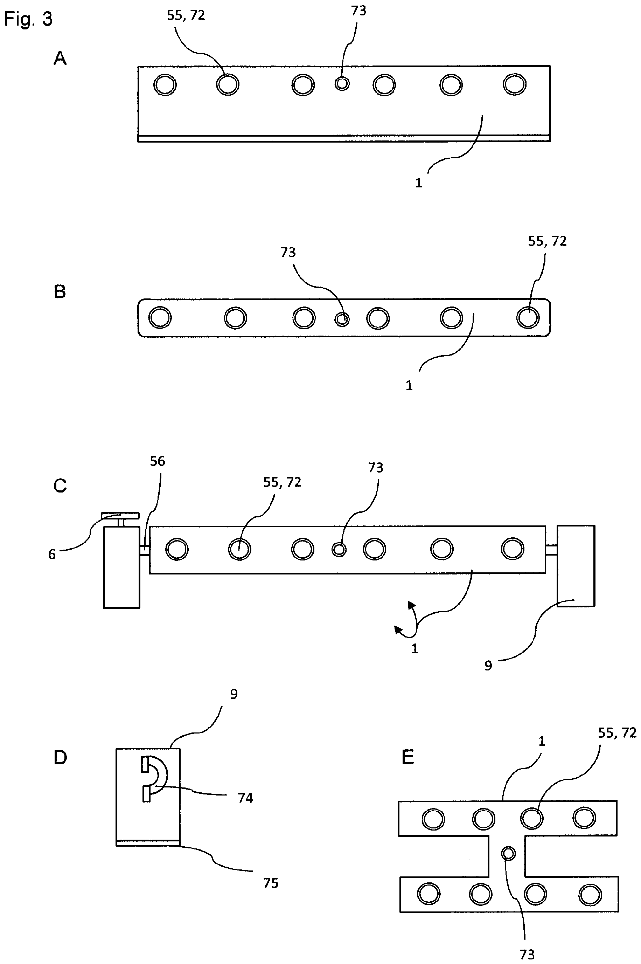

Figur 3 zeigt unterschiedliche Halteflächen und Teile zum Anbringen von Vorrichtungsadaptern. Alle aufgeführten Darstellungen **A** bis **C** und **E** besitzen Durchbrüche die auf die Vorrichtungsdurchbrüche von Siebkörben, Gestellen oder Waschgutträger exakt abgestimmt sind, insbesondere um eine Standardisierung zwischen den Vorrichten zu erziehlen. Die Haltefläche sind vorzugsweise abdichtbare Flächen. Alternativ können diese auch an der Vorderseite zusätzliche Dichtflächen, Materialerhebungen oder Materialvertiefungen oder eine Materialnute oder ähnliches zur besseren Dichtung besitzen oder hervorstehende Gewindehülsen aufweisen. Die Darstellung **A** zeigt eine Vorderansicht mit einer abgewinkelten Adapterhalterung **1** (bzw. Schiene) mit Durchbrüchen **55** die auch Gewindelöcher **72** zum Festlegen von Vorrichtungsadaptern sein können. Der Halterungswinkel **1** besitzt zentral in der Mitte ein zusätzliches Haltegewinde **73,** z. B. zum Festlegen von Haltewellen eines Mediumverteilers. Diese Gewinde **73** können auch mehrgängig sein, um das Anbringen zu erleichtern. Die anschließende Darstellung **B.** zeit eine einfachen Haltestreifen oder Leiste **1.** Darstellung **C.** besitzt ebenfalls Durchbrüche **55,** Adaptergewinde **72** sowie ein Adaptergewinde **73.** Über seitliche Stifte **56** und beabstandete Halteblöcke oder Halteteile **9** ist die drehbare Adapterhaltevorrichtung mit z. B. einem Siebkorbboden verbunden. Über zumindest einer Rändelschraube **6,** die oben oder seitlich angeordnet sein kann, wird die Halteleiste **1** der Vorrichtung in unterschiedlichen Winkeln festlegbar und über die seitlichen Stifte **56** drehbar (mit einem Pfeil angedeutet). Die Darstellung **D** zeigt ein Haltescharnier mit einer Haltenute und einem Fußteil **75** zum Festlegen an einem Siebkorbboden. Über die Haltenute **74** wird der drehbare Freiheitsgrad eines Adapterhalteteils definiert. Eine Rändelschraube wird hier nicht zur Festlegung benötigt. Abbildung **E** zeigt eine flächige Ausführung einer Haltefläche **1** mit geometrisch angeordneten Durchbrüchen **55** oder Adaptergewinden **72.** Auch hier befindet sich Zentral ein Haltegewinde **73.** Diese Halteplatte **E** kann bevorzugt auch bei Drahtsiebkörben und für den Einsatz von Flachmediensteckern eingesetzt werden.

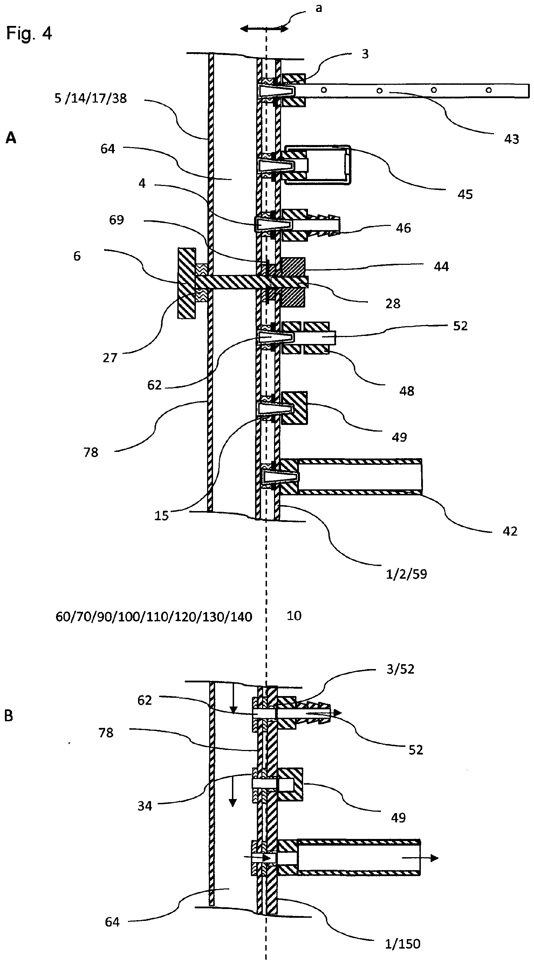

Figur 4. zeigt zwei in Schnittdarstellung aufeinander abgestimmte Adapteranordnung. Die Darstellung **A** zeigt im Schnitt, wie zwei unterschiedliche Adapteranordnungen **3** und **4** zu einer Reinigungsvorrichtung zusammengeführt sind. Dabei ist ein Rohr **5** (alternativ auch ein Hauptmediumverteiler **14,** eine Medienwand **17** oder ein Medienstecker **38**) eines Mediumverteilers gezeigt. Über die Vorrichtungswände **78** ist eine Rändelschraube oder ein Fixierungsteil **6** in Form einer Haltewelle **28** durch den Mediumraum **64** geführt und mit einer Siebkorbwand **2** oder eine Halteplatte **1** oder einem Siebkorbboden **59** verschraubt. Zur besseren Handhabung besitzt das Fixierungsteil **6** am Wellenschaft **28** beabstandete Dichtungen **27** und einen Sicherungsring **69** zur drehbaren Festlegung der Welle **28.** Zur Verschraubung des Fixierungsteils **6** besitzt z. B. die Siebkorbwand **2** ein Halteadapter bzw. eine über eine Mutter fixierten Einschraubadapter **44.** Über die eingesteckten Steckadapter **4** oder deren Anordnung am Mediumverteiler kann in die einsteckbaren Siebkorbadapterteile **3** Reinigungsmedium über die verbundenen Kanäle der Adapter weitergeleitet werden. Die Verschraubung mit einem elastischen Anpresselement **27** und die Dichtungen **15** der Steckadapter **4** sorgen für eine ausreichende Dichtigkeit der Verbindung und auch dafür, dass diese sich nicht zu leicht von selbst löst. Die steckbaren Adapterteile **3** besitzen vorderseitig eine gleich gestaltete Adapteröffnung zum Einführen z. B. von Steckadapter eines Mediumverteilers. Der rückseitige Adapterteil kann als Verschlussadapter **49,** als Luer Lock männlich Adapter **48,** als Schlauchadapter **46,** als Spüllanzenadapter mit seitlichen Öffnungen **43,** als Spülhülsenadapter **42** oder als Kappenadapter **45** ausgeführt sein. Der **Pfeil a** und die gestrichelte Linie symbolisieren, dass die Reinigungsvorrichtung **10** in zwei eigenständige Spülvorrichtungen teilbar ist. Teilbar in einem Mediumverteiler (eine Verteilerleiste **100, 110, 120** oder ein Mediumstecker oder Verteilerbox **130** oder **140** oder einen zylindrischen Mediumverteiler **90**) und beispielsweise in Ausgestaltung eines Siebkorbs **20** oder **150,** bei dem ein Adapterteil **3** mit einer Siebkorbwand **2** oder mit einem Siebkorbboden **59** oder mit einer Halteleiste **1** oder mit einer beweglichen Registerleiste **140** hergestellt ist. Alternativ bildet die Halteleiste mit Adaptern auch als eine eigenständig nutzbare Spülvorrichtung aus. In einer anderen Schnittdarstellung **B** sind unterschiedliche Adapter zu einer Reinigungsvorrichtung verbunden. Dabei Hält eine vordere oder alternativ auch eine hintere Haltefläche oder Halteplatte **1** die Adapterteile oder Adapter **3, 49** und **42** und dichtet über Dichtelemente **34** die eingebrachten Steckadapter **4** eines Medienverteilers. Die Vorrichtung zeigt, dass bei der Kopplung der Kanal **62** des Dichtadapters mit einem Kanal **52** mit dem gegenüberliegenden Produktregisteradapter durchgängig verbunden ist. Der Mediumraum **64** verteilt dabei die zugeleiteten Medien (dargestellt durch Pfeile) in die verbundenen offenen Adapterkanäle **52** und **62.**

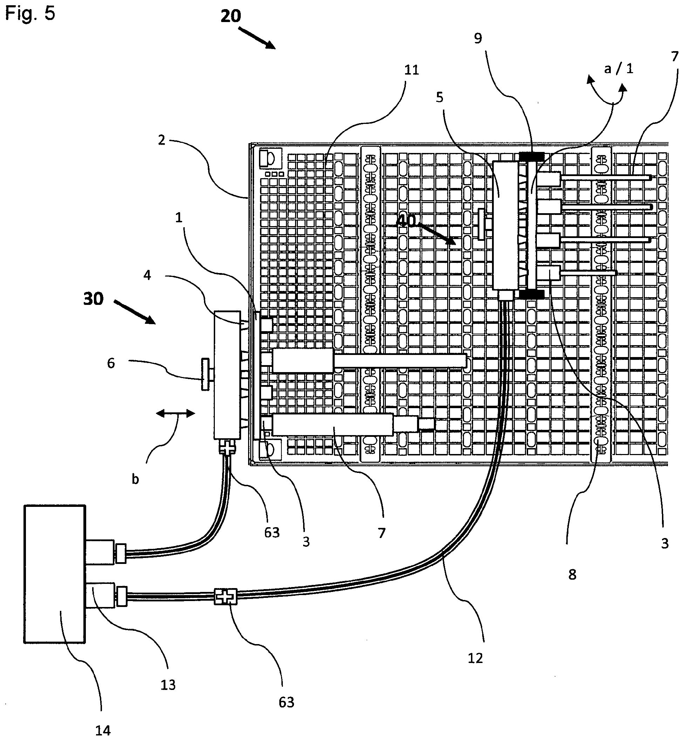

Figur 5. zeigt in einer Draufsicht eine Siebkorbreinigungsvorrichtung mit teilbarenzwei Reinigungsvorrichtungen die über biegsame Zuleitungen mit Reinigungsflüssigkeit versorgt werden. Die Siebkorbreinigungsvorrichtung **20** enthält zwei gekoppelte Reinigungsvorrichtungen **30** und **40.** Darstellung **30** zeigt Steckadapter **4** (Luer männlich Adapter) die in Adapterteile **3** stecken. Letztere Adapter sind an einer Siebkorbwand **2** festgelegt. Eine Haltefläche oder ein Halteteil **1** nimmt dabei die Adapterteile **3** auf. Die Medieneinleitung erfolgt über Schläuche **12** als Zuleitung. Die Schläuche **12** sind dabei vorzugsweise über selbstverschließbare Maschinenadapter **13** und geeigneten Steckadaptern und/oder Medienhähne **63** (Verschlussvorrichtung) an einer Hauptmediumleitung **14** der Maschine angeschlossen. Auch die Medienvorrichtung kann einen Hahn **63** oder Ventil aufweisen. Alternativ kann hier auch eine Mediumwand diese Aufgabe übernehmen. Damit die Registerleiste **1** drehbar (dargestellt durch den **Pfeil a**) ist, sind seitlich Halteteile **9** vorhanden, die mit dem Siebkorbraster **11** von unten verschraubt sind. Darstellung **40** zeigt eine eigenständige und bewegbare Haltefläche **1** die mit einem Siebkorbboden und/oder einer Seitenwand **2** verschraubt ist. Die Registerleiste **1** kann über einen Haltewinkel mit dem Siebkorbbodengitter **11** verbunden sein. Im Siebkorb **20** werden medizinische Produkte **7** wie z. B. Bohrer ohne Kanäle über Adapter (z. B. selbstverschließbare Kappenadapter) zur besseren Spitzenreinigung gehalten. Das wandständige Halteteil oder Haltefläche **1** besitzt eine Adapteranordnung **3,** an der medizinische Produkte **7** (hier Bohrer, Bohrwellen etc.) mit zumindest einem Kanal angeschlossen sind. Die eingelagerten medizinischen Produkte und Instrumente **7** werden im Siebkorb **20** über Lamellenhalterungsstreifen **8** oder alternativ über Noppenstreifen etc. positioniert. Zur sicheren Befestigung der Mediumverteiler besitzen diese eine Rändelschraube **6** oder ähnliches, die über das Rohrlumen **5** mit einer Siebkorbwand oder der Registerleiste lösbar verbunden ist. **Pfeil b** verdeutlicht die Beweglichkeit des Mediumverteilers. Leisten **1** bieten den Vorteil einer qualitativen und passgenauen Mediumkopplung, so dass eventuell auf eine zusätzliche Dichtung der Steckadapter verzichtet werden kann.

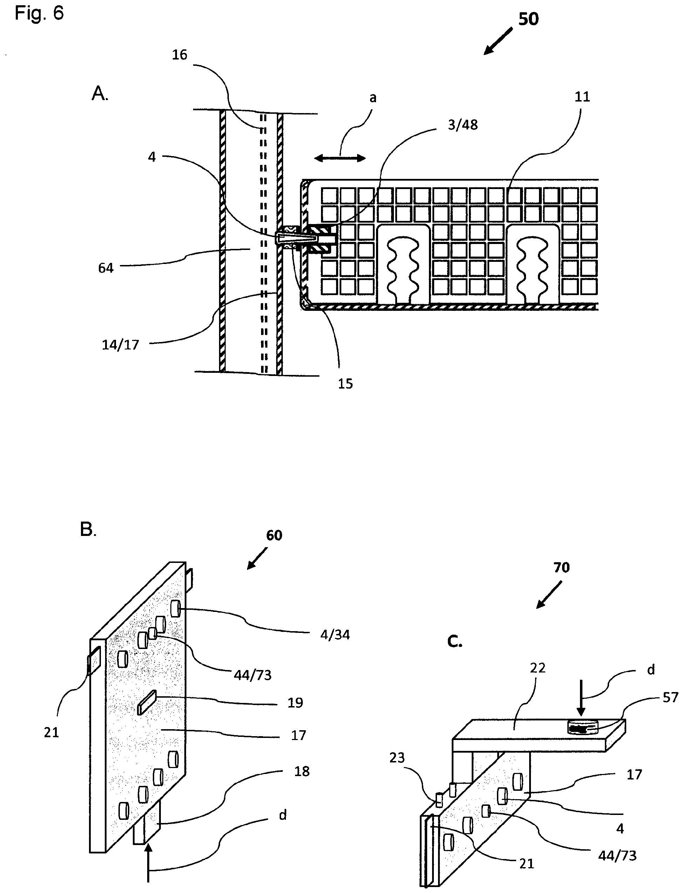

Figur 6 zeigt eine Reinigungsvorrichtung in Schnittdarstellung bestehend aus einem Siebkorb mit unterschiedlich gestalteten Medienwänden eines Gerätewagens. Die Darstellung **A.** zeigt eine Reinigungsvorrichtung **50** mit einem Mediumraum **64.** Am Siebkorbgitter **11** eines Siebkorbs befinden sich innenliegend, zumindest steckbare Adapterteile **3.** Der Produktadapterteil **3** mit einem Luer Lock männlich Anschlussadapter **48,** ist mit einer Hauptmedienleitung **14** oder einer Medienwand **60** oder **70** eines Gerätewagens oder eines Siebkorbs in einer Reinigungsmaschine über ein Steckadapter **4** lösbar verbunden. Alternativ kann die Hauptmedienleitung **14** auch eine doppelwandige Geräterückwand sein. Der **Pfeil a** zeigt an, dass der Siebkorb über den Steckadapter **4** in Verbindung mit einem Dichtteil **15** mit einer Medienhauptleitung **14** lösbar und dichtend verbunden ist. Zur Filterung des Mediums kann sich z. B. in einer Medienwand **60** oder **70** bzw. Hauptmedienleitung **14** bzw. ein flächiger Filter **16** befinden. Darüber hinaus können sich Halteteile in Form eines Fixierungsadapter **44** oder Haltegewinde **73** an der Medium-Innenwandung **17** (siehe Abbildung 60 und 70) befinden, damit beispielsweise hier Mediumverteiler (Spülleisten, Spülzylinder etc.) oder rüber größere Ausgänge **19** Dreharme oder sonstige Vorrichtungen festlegbar sind. Abbildung **B** zeigt eine ausgestaltete Medienwand **60,** die in einem Etagenwagen eingebracht werden kann. Die Medieneinleitung **18** kann hier von unten, aber auch von oben oder von der Seite über z. B. einen steckbaren Zulauf erfolgen. An der Innenwand **17** der Vorrichtung befinden sich Steckadapter **4** und/oder Dichtadapter zur Medienausleitung. Seitlich kann die Wandung **17** zwei Etagen eines Etagenwagens auskleiden und hier auch größere Medienausleitungen **19** oder andere Adapter in der Fläche bereitstellen. Der **Pfeil d** zeigt an, dass beispielsweise eine Medienzuführung von unten erfolgen kann. Um die Medienwand zu fixieren, können seitliche Halterungen **21** angebracht sein. Vorteil dieser Medienwand **60** ist, dass diese sich bereits durch Ihre Gewichtskraft hält. Darüber hinaus kann die Medienwand **60** weitere Ausgänge **19** für Spülverteiler (Dreharme etc.) aufweisen. Die Darstellung **C** zeigt eine Medienwand **70,** die in der oberen Ebene eingebracht ist. Medienausleitungen befinden sich an der Innenwand **17** sowie auf der Oberseite der Medienwand **23.** Die Medienzuführung erfolgt hier z. B. von oben über eine Anpresskopplung **57** o. ä (siehe **Pfeil d**)**.** Unter der Medienzuführung kann zusätzlich auch ein Dreharm angebracht sein. Darüber hinaus, kann die Vorrichtung in Abbildung C. auch links und rechts eine Medienwand mit Reinigungslösung versorgen (symmetrischer Aufbau). Medienwände **60** oder **70** oder in anderer Ausgestaltung, können mobil oder fest in einem Etagenwagen über Haltelamellen **21** z. B. über U-Schienen gehalten sein. Alternativ kann die Medienwand 70 auch nur eine Hauptmedienleitung sein.

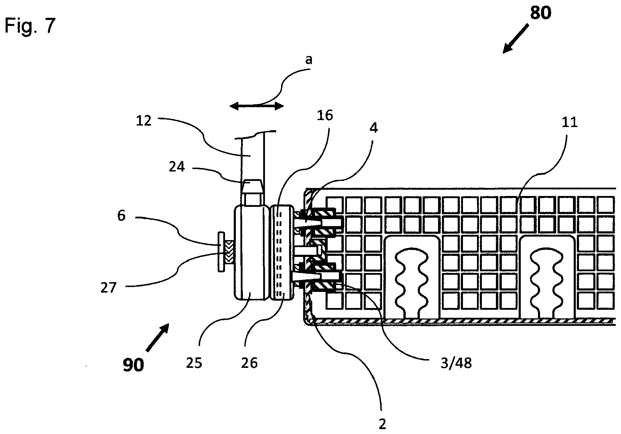

Figur 7 zeigt eine Reinigungsvorrichtung mit einem Siebkorb und einem zylindrischen Mediumverteiler. Dargestellt ist eine Reinigungsvorrichtung **80** mit einem Siebkorb und einem zweiteiligen zylindrischen Mediumverteiler **90** (Spülmedienzylinderstecker). Die Siebkorbvorrichtungen **80** und **90** bilden die Reinigungsvorrichtung. Der zylindrische Mediumverteiler **90** bestehend aus einem Medienanschlussteil **25** und einem Medienverteilkopf **26.** An diesem Kopf **26** befinden sich die Steckadapter **4.** Die Festlegung des Spülzylindersteckers **90** wird über eine Rändelschraube **6** und einer vorzugsweise elastischen Hülse **27** oder ähnlichem unterstützt. Dabei wird eine Gewindeschraube durch das Innenlumen der Vorrichtung **90** geführt und über diese mit der Siebkorbwand **2** verschraubt. Der Spülsiebkorb **80** besitzt seitliche Formelemente und im Inneren an einer Seitenwand geometrisch und flächig angeordnete Adapter, die ein Adapter **3** mit einer steckbaren Seite und auf der anderen Seite z. B. einen Luer Lock Adapter ausbilden. Die Medienzuführung in den zylindrischen Mediumstecker **90** erfolgt hier seitlich über einen Medienanschluss **24** in Verbindung mit einem Medienzuführschlauch **12.** Über einen innenliegenden Filter **16** des Spülmediumsteckers **90** kann Reinigungsmedium gefiltert werden.

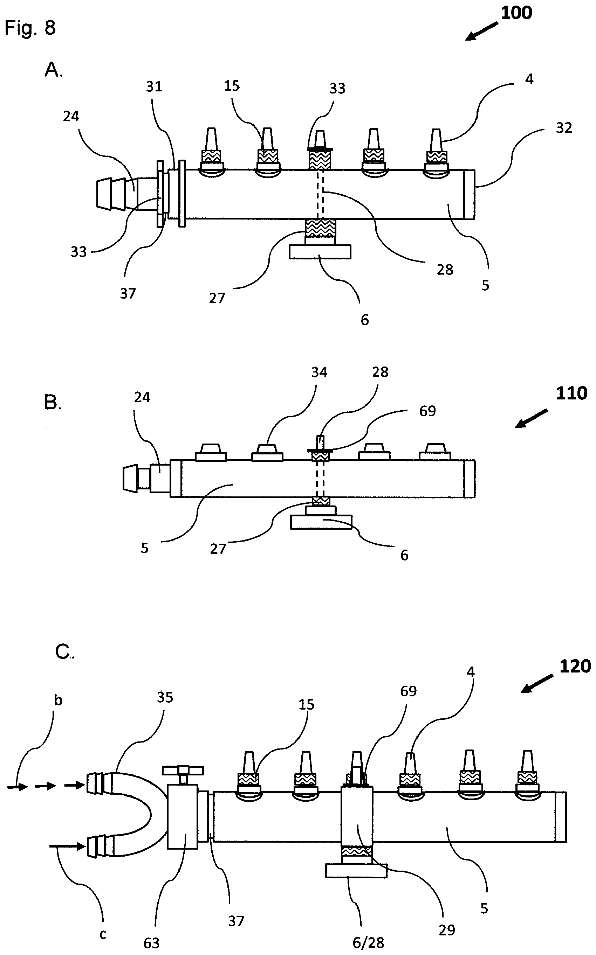

Figur 8 zeigt unterschiedliche mobile Rohrverteiler (Spülleisten bzw. Spülmodule). In **A** ist ein Mediumverteiler als Mediumstecker **100** (bzw. Spülleistenstecker) dargestellt. Dieser besteht aus einem Medienrohr **5** mit einer endständigen Verschlusskappe **32,** einem geschraubten Steckadaptern **4** (konisch oder gerade zulaufend) mit einem Dichtelement **15** und einem vorderen Medienanschluss-Schlauchadapter **24.** Der Medienstecker **100** besitzt ein vorderes ovales Ansatzelement **33** mit einer dahinterliegenden Nute **37.** Der Schlauchadapter **24** ist entfernbar und kann durch einen anderen Adapter ersetzt werden. Über die Nute **37** in Verbindung mit einem geeigneten elastischem Anpresselement auf dem Ansatz **31** kann das Spülrohr **5** bzw. der Spülmediumstecker **100** mit einer geeigneten Siebkorbwand lösbar verbunden werden. Damit der Mediumstecker an einer Wandung fixierbar ist, befindet sich ein Halteblock **29** am Spülrohr 5. Über den Halteblock **29** kann mit einer Rändelschaube **6** vorzugsweise in Verbindung mit einem elastischen Anpresselement **27** auch in Ausgestaltung eines Dichtelementes bzw. elastischen Hülse (z. B. einer Silikonscheibe, Silikonmanschette etc.) über eine Gewindestange oder eine Haltestange **28,** die Vorrichtung lösbar mit Adapter, einer Wandung oder einem Boden verbunden werden. Das Anpresselement **27** oder die Dichtelemente **15** dienen dazu, dass die Vorrichtung sich leichter von Hand lösen lässt. In Abbildung **B** ist in Seitenansicht ein Spülmedien-Verteildichter **110** mit einer Fixierungsschraube **6,** die durch den Innenraum geführt ist, dargestellt. Der Spülmedien-Verteildichter **110** besteht aus einem rechteckigen Medienrohr **5** mit einem endständigen Verschlussteil, einem vorderen Schlauchanschluss **24** und einem in Reihe angeordneten Dichtadaptern **34.** Die Vorrichtung kann, z. B. über eine Rändelschraube **6** mit einem elastischen Anpresselement und einer durchgehenden Haltewelle 28 aufweisen. Diese kann mit einem vorderen Gewinde an ein Halteteil (Registerleiste), Wandung oder einem Boden lösbar angeschraubt werden. Über den Spülmedien-Verteildichter **110** werden die jeweiligen Anschlussadapter z. B. an der Siebkorbwand durch einen Dichtadapter **34** der Vorrichtung überdeckt und so abgedichtet. In einer Weiterführung ist in **C** ein Mediumstecker **120** (Spülmedienleistenstecker) gezeigt. Dieser besteht aus einem Medienrohr **5** mit einem endständigen Verschlussteil, einem geschraubten Steckadaptern **4** (Adapter konisch oder gerade zulaufend) mit jeweils einem Dichtelement **15** und einem vorderen U-förmigen Medienanschluss-Schlauchadapter **35.** Der Aufbau entspricht im wesentlichem der Vorrichtung **100,** jedoch mit dem Vorteil, dass diese über einen Hahn **63** verschließbar oder hierüber ein Medienmix organisiert wird. Ferner können über den U-förmigen Anschluss **35** unterschiedliche oder eine doppelte Medienmenge in die Vorrichtung eingebracht werden. Schematisch ist durch die **Pfeile b** symbolisiert, dass hier das Medium gepulst eingebracht wird. Am gegenüberliegenden Arm hingegen erfolgt ein nicht gepulster Medieneinstrom. Es können gleiche Aufbereitungsmedien (wässrige Reinigungslösung **Pfeil c**) oder unterschiedliche Medien (Dampf, Luft in Verbindung mit wässrigem Medium) eingeleitet werden. Die Anzahl der Adapter am Medienstecker richtig sich auch nach der Anzahl der möglichen Anschlussmöglichkeiten am Siebkorb.

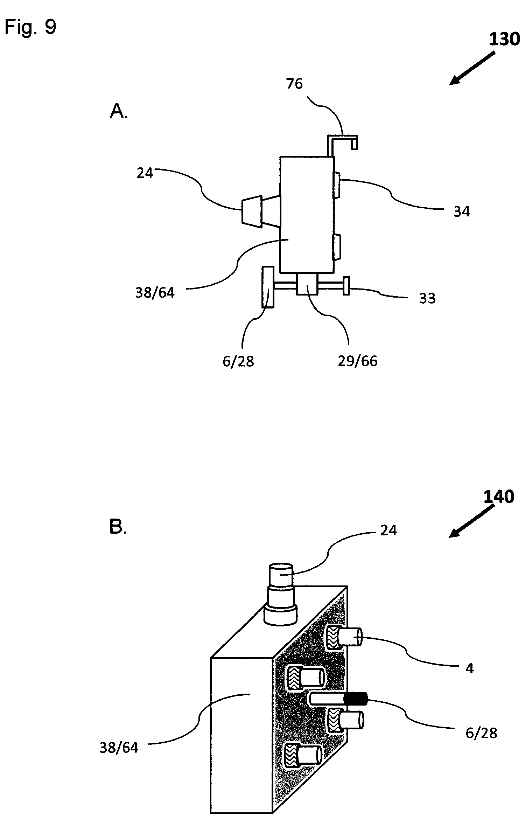

Figur 9. zeigt unterschiedliche eckige Mediumstecker. Abbildung **A** zeigt einen eckigen Mediumstecker bzw. Mediumstecker **130** mit einem eckigen Mediumraum **64.** Der Spülmodulkörper **38** wird über ein oberes Eingreifteil **76** in eine Siebkorbwand oder an eine Medienwand gehängt und z. B. von unten oder seitlich über eine drehbare Fixierungsteil **6** mit einer Haltestange oder -welle **28** mit einem Siebkorbdurchbruch über eine vorderes Ansatzteil **33** verbunden. Der Mediumeintritt erfolgt von hinten über einen Medien- oder Steckadapter **24** und von vorne beispielsweise über Dichtadapter **34.** Die Haltewelle besitzt auf dem Schaft **28** ein Gewinde oder ist anders mit einem Block **29** oder mit einer z. B. Rändelschraubenhalterung **66** verbunden. Derartige Vorrichtungen können auch zwei Halteteil **76** aufweise und so dichtend über die Dichtadapter eingeschoben werden. In **B.** ist ein weiteres quadratisches Spülmodul bzw. ein Medienstecker **140** (bzw. eine Medienverteilerbox) mit einem quadratischen oder rechteckigen Spülmodulkörper **38** mit einem Medienraum **64** gezeigt. Über eine Haltewelle **6** mit einen heraustretenden Schaft **28** mit einem vorderen Gewindeansatz und über die Steckadapter **4** z. B. wird das Modul mit einer Siebkorbwand verbunden. Von oben erfolgt die Medienzuführung, z. B. über einen Schlauchadapter **24.** Alternativ kann die Medienzuführung auch seitlich erfolgen oder es können weitere Medienanschlussadapter oder im Inneren des Körpers ein Filter vorhanden sein.

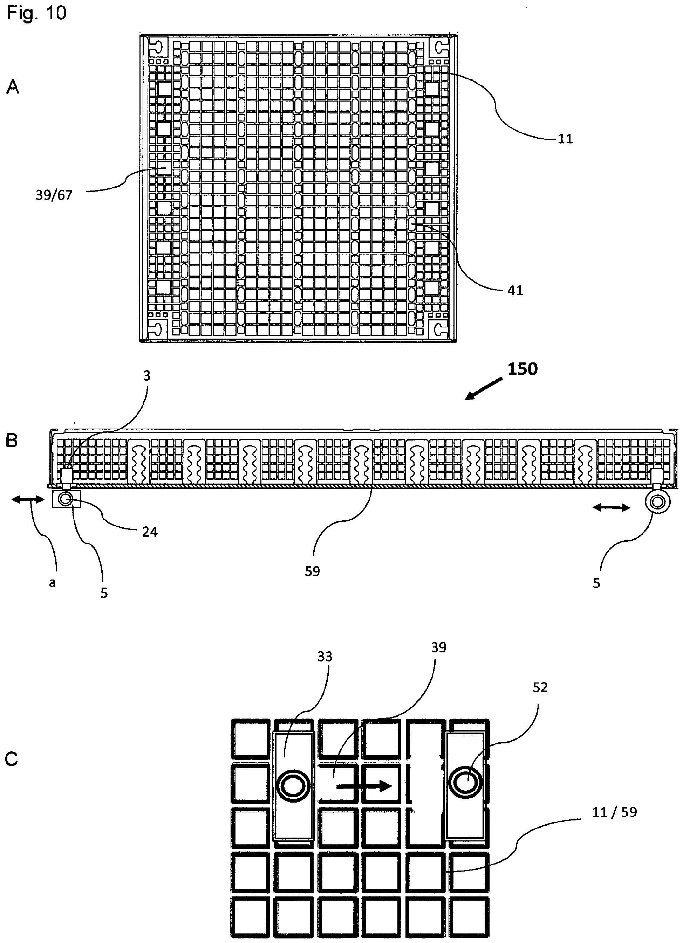

Figur 10. zeigt Siebgitter mit im Boden angeordneten Mediumverteilern und unterschiedliche Durchbruchsrastern. **A** zeigt ein Siebkorbgitter **11** von oben. Der Siebkorbboden besitzt ein geometrisch angeordnetes Durchbruchsgitter **11.** Hier sind vorzugsweise ovale und große quadratische Durchbrüche **41** enthalten sowie größere kreuzartige Durchbrüche **39** (siehe Abbildung C). In diese können Mediumverteiler (rund oder eckig) von unten oder Adapter eingebracht werden. Abbildung **B** zeigt eine Reinigungsvorrichtung **150** mit unten angeordenten Medienverteilern. Links ist ein rechteckiges Spülrohr **5** und rechts ein rundes Rohr **5** gezeigt. Über diese Rohre **5** werden von unten über die Durchbrüche im Siebgitter des Siebkorbbodens **59** die Steckadapter der Mediumverteiler ins Siebkorbinnere eingebracht oder die am Siebkorbboden angebrachten Adapter **3** angeschlossen und mit Medien versorgt. Über einen Mediumanschluss **24** (Schlauchadapter) werden die Medienverteiler (siehe hierzu Figur 4) mit Reinigungslösung etc. versorgt. Die **Pfeile a** deuten wiederum nur an, dass die Medienverteiler lösbar mit dem Siebkorb verbunden werden können. Die Abbildung **C.** zeigt ein Siebkorbgitter eines Siebkorbbodens **59** mit vergrößerten Durchbrechungen **39,** um durch Schieben oder Drehen der Adapter-Steckseite **33** einfach zu lösen. Alternativ können über die Durchbrüche **39** oder ähnliche Durchbrüche auch Mediumverteilerrohre **5** über die Steckadapter mit einer rechteckigen Adaptersteckseite bzw. einem Ansatzteil **33** fixiert werden (durch den Pfeil verdeutlicht).

### Bezugszeichenliste (Auszug)

1 Halteteil oder -fläche für Adapter, Wellen, Schrauben in Ausgestaltung einer Leiste, Schiene oder einem Streifen
2 Siebkorbwand mit Durchbrüchen
3 Vorrichtungsadapteranordnung und Korbadapter (steckbarer Adapterteil oder Adapteransatzteil)
4 Verteileradapteranordnung und Mediumverteileradapter (Steckadapter z. B. Luer männlich oder einsteckbarer Adapterteil eines Spülmoduls)
5 Rohr (quadratisch, rechteckig oder rund)
6 Drehbares Fixierungsteil mit einem Schaft in Ausgestaltung einer Welle, Stange, Stift oder das diese eine Rändelschraube oder nur eine Schraube ist
7 Spülprodukt, medizinisches Gerät oder Produkt mit (Hohlraumprodukt) oder ohne Kanal
8 Lamellenhalterungsstreifen oder Noppenband
9 Halterungsteil oder Halteleiste, Halteschiene oder Halterungswinkel
11 Siebkorbgitter oder Vorrichtungsdurchbrüche im Boden oder an den Wänden
12 Zuleitung, Schlauchleitung, biegbare Leitung o. ä.
14 Medien- oder Hauptmediumleitung
15 Dichtung für Anschlussadapter
17 Vordere Medienwand
18 Medieneinleitung
27 Elastisches Halte-, Anpress- oder Dichtelement
28 Schaft, Haltewelle, Haltestange, Haltestift o. ä. eines drehbaren Fixierungsteils
29 Block als Halteteil für Fixierungsschrauben etc.
33 Ansatzteil (vorzugsweise eckig)
34 Selbstdichtender Adapter oder elastischer Dichtadapterteil für einen Mediumverteiler
35 U-förmiger Medienanschluss oder Medienzuleitung
37 Nute zur Festlegung eines Adapters
42 Spülhülse oder Adapterteil
43 Spüllanze mit seitlichen Löchern
44 Halteteil und Fixierungsadapter (Vorrichtungsadapter) zum Festlegen von Wellen, Schrauben etc.
45 Kappenadapter (Anschlussadapter oder Adapterteil)
46 Schlauchadapter (Anschlussadapter oder Adapterteil)
48 Luer Lock männlich Adapter (Anschlussadapter oder Adapterteil)
49 Verschlussadapter (Anschlussadapter oder Adapterteil)
51 Luer Lock weiblich Adapter (Anschlussadapter oder Adapterteil)
52 Adapterkanal und Kanalöffnung, steckbar und/oder dichtend oder besitzt ein durchgehendes Innengewinde
54 flache Schraubenmutter mit Spezialgewinde und vordere plane Dichtfläche, Halteteil
57 Anpresskopplung oder Kopplungsteil eines Reinigungs- oder Etagenwagens
59 Siebkorbboden oder Gitterboden
62 Adapterkanal eines Steck- oder Dichtadapters
63 Hahn oder Ventil (z. B. Einweg- oder Mehrwegahn)
64 runder oder eckiger Mediumraum zur Mediumverteilung (flüssig und/oder gasförmig) 67 Adapterdurchbrüche einer Wandung oder eines Bodens
69 Sicherungsring oder Wellenring
73 Gewinde eines Halteteil oder eines Mediumverteilers zum Festlegen von Medienverteiler und -stecker
78 Vorrichtungswand (Siebkorbwand, Wand eines runden oder eckigen Mediumverteilerraums etc.)

## Patentansprüche

1. Reinigungsvorrichtung zur Hohlrauminstrumentenreinigung für die medizinische Produktreinigung und für den OP, aufweisen einen Siebkorb mit Durchbrüchen und Korbadapter (3) und einem Mediumverteiler mit Mediumverteileradapter (4, 34) und Mediumraum (64) wobei die Korbadapter und die Mediumverteileradapter aufeinander abgestimmt sind, **dadurch gekennzeichnet, dass** zumindest die Reinigungsvorrichtung ein Fixierungsteil (6) aufweist, wobei das Fixierungsteil (6) eine von Hand drehbare Haltewelle (28), -stift, -stange oder -schraube ist und diese außen an einer Mediumwandung (17, 78) des Mediumverteilers geführt oder durch die Wandung (17, 78) des Mediumraums (64) des Mediumverteilers geführt und gehalten wird damit das Fixierungsteil (6) über eine von Hand steuerbare Anpresskraft den Mediumverteiler mit einer Siebkorbwandung (2), einem Siebkorbboden (59) oder an einer dieser befestigten Haltefläche (1) zumindest festlegbar ist und dabei eine Kopplung koppelbarer Korbadapter (3) mit Mediumverteileradapter (4, 34) so erfolgt, dass dabei Adapterkanäle (52, 62) miteinander gekoppelt sind.

2. Reinigungsvorrichtung nach Anspruch 1, **dadurch gekennzeichnet, dass** zumindest die Durchbrüche (41) oder das Durchbruchsgitter (11) der Reinigungsvorrichtung eine definierte Adapter- und Halterungsanordnung und deren Ausrichtung vorgibt oder mitbestimmt und in seiner Relation zum zu reinigenden Kanaldurchmesser eine Durchbruchsgröße zur Verfügung stellt, damit eine sichere Kanalreinigung erfolgen kann und wobei die Reinigungsvorrichtung mit Halteteile vorgesehen ist, so dass wenn vom Durchbruchsgitter (11) abweichende oder vergrößerte Durchbrüche (41) in der Vorrichtung vorhanden sind diese mit Halteteile (1) überbrückt werden, um Korbadapter (24, 45, 46, 48, 49, 51) und Halteteile (1, 44) festzulegen oder um über die Durchbrüche Adapter (4, 34) des Mediumverteilers von außen über Durchbrüche (67) in die Vorrichtung eingebracht werden können.

3. Reinigungsvorrichtung nach Anspruch 1 bis 2, **dadurch gekennzeichnet, dass** der Siebkorb der Vorrichtung ein chirurgisches Set an medizinischen Instrumenten für einen medizinischen Eingriff zur Anwendung im OP enthält und der Mediumverteiler zumindest einem von Hand bewegbareren und eigenständig nutzbarer Mediumverteiler (90, 100, 110, 120, 130, 140) ist und der in einer Reinigungsmaschine oder in einem Ultraschallbad oder außerhalb dieser Geräte mit Medium versorgt wird.

4. Reinigungsvorrichtung nach einem der vorherigen Ansprüche, **dadurch gekennzeichnet, dass** die Siebkörbe (10, 20, 150) mit Korbadapter (24, 45, 46, 48, 49, 51) und die Mediumverteiler (90, 100, 110, 120, 130, 140) mit Mediumverteileradapter (4, 34) eigenständige Reinigungsvorrichtung sind und die Mediumverteiler zumindest über feste Zuleitungen (18, 22) oder über biegbare Zuleitung (12) mit Reinigungsmedium versorgt werden.

5. Reinigungsvorrichtung nach einem der vorherigen Ansprüche, aufweisend einen Fixierungsadapter, wobei das Haltegewinde (73) zumindest der Fixierungsadapter (44) ein ein- oder mehrgängiges Gewinde mit einer durchbruchsbezogenen Ausformung (68) nach dem Gewinde aufweist, um das Verdrehen des Halteteils oder der Korbadapter in der Vorrichtung zu unterbinden und wenn das Halteteil (1) ein Streifen oder eine Schiene ist und über einen Haltewinkel (9) als ein drehbares Halteteil (1) die Korbadapter (24, 45, 46, 48, 49, 51) festlegt und hieran ein Mediumverteiler festsetzbar ist.

6. Reinigungsvorrichtung nach einem der einem der vorherigen Ansprüche, **dadurch gekennzeichnet, dass** eine Kopplung unterschiedlicher Korbadapter und Mediumverteileradapter (3, 4) erfolgt und dabei die Haltewelle, der Halteschaft oder der Haltestift (28) ein vorderes Gewinde oder ein Ansatzteil (33) zumindest das Fixierungsteil (6) mit Dichtelemente (27) und/oder einer Haltenute für Springringe (69) aufweist und dabei durch einen Block (29) oder ein Halteteil (66, 76) geführt sein kann.

7. Reinigungsvorrichtung nach einem der vorherigen Ansprüche, **dadurch gekennzeichnet, dass** die Korbadapter und Mediumverteileradapter (3, 4) in Reihe, flächig und geometrisch angeordnet sind und die Steckteile (4) Dichtungen (15) aufweisen oder selbstdichtende Mediumverteileradapter (34) sind, um Adapteröffnungen (52) abzudichten und dabei die Steckadapter (4) und/oder die selbstdichtenden Mediumverteileradapter (34) die Kopplung unterstützen.

8. Reinigungsvorrichtung nach einem der vorherigen Ansprüche, **dadurch gekennzeichnet, dass** der Mediumraum (64) zumindest in Ausgestaltung eines Rohrs (5), eines Zylinders (16) oder eines eckigen Mediumraums (5, 38, 60, 70) mit zumindest einem biegsamen Medienzulauf (12) oder einen feststehenden Medienzulauf (14, 18, 22) aufweist oder zusätzlich über einen Hahn (63) verschließbar ist, wobei zusätzlich über einen U-förmigen Adapter (35) unterschiedliche Medien einleitbar und mischbar sind und dieser zusätzlich einen Filter (16) enthält.

9. Reinigungsvorrichtung nach einem der vorherigen Ansprüche, **dadurch gekennzeichnet, dass** medizinische Produkte (7) in dem Siebkorb oder an eine Haltefläche (1) der Reinigungsvorrichtung in Ausgestaltung über eine Adapterleiste oder einer Halteplatte in dem Siebkorb angeschlossen oder gehalten sind und zumindest damit eine Lagerungsstabilisierung und zusätzlich über Kennzeichnungen die Einsortierung und das Instrumenten- und Anschlussmanagement im Siebkorb unterstützt, wobei zusätzlich elektronische Sensoren oder RFID-Systeme zumindest am Korbadapter vorhanden sind.

10. Reinigungsvorrichtung nach einem der vorangegangenen Ansprüche, **dadurch gekennzeichnet, dass** die enthaltenden Korbadapter direkt mit einer Halteplatte (1) oder mit einer vorderen abdichtbaren Mutter (54) an einer Siebkorbwand (2) oder an einem Siebkorbboden (59) verschraubt und über den Boden drehbar angeordnet sein können, um diese individuell anzuordnen und über ein verschlossenen Korbadapter (49) an Stellen nicht genutzter Durchbrüche einen nicht genutzten Medienaustritt verhindern.

11. Reinigungsvorrichtung nach einem der vorangegangenen Ansprüche, **dadurch gekennzeichnet, dass** diese mehrteilige Korbadapter oder einschraubbare Korbadapter aufweisen, die vorderseitig einen geraden oder konischen Kanal (52) und eine kaum hervorstehende abdichtbare Kanalöffnung aufweisen und rückseitig als Anschlussteil einen Schlauchadapter (46), Luer Lock weiblich (51), Luer Lock männlich Adapter (48), Steckadapter (4), Kappenadapter (45), Verteilerlanzen und - düsen (43), Verschlussadapter (49), Hülsenadapter (42) und/oder Fixierungsadapter (44) besitzt.

12. Reinigungsvorrichtung nach einem der vorangegangenen Ansprüche, **dadurch gekennzeichnet**, das zumindest die Festlegung der Korbadapter und deren Adapteranordnung (3, 4) an Durchbrüche einer Wandung (2), an einem Boden (59) des Siebkorbs über eine Nute (37) und einem Anpresselement (27) und/oder einem Wellenring durch eine Rückstellkraft eines elastischen Anpresselements (27) und durch einen quadratischen Adapteransatz unterstützt ist.

13. Reinigungsverfahren, für die maschinelle medizinische Produktreinigung und für den OP, mit einer Reinigungsvorrichtung nach einem der obigen Ansprüche **dadurch gekennzeichnet, dass** im ersten Schritt medizinische Produkte in dem Siebkorb an die Korbadapter oder über Schläuche an die Korbadapter angeschlossenen werden, um im zweiten Schritt von Hand der Mediumverteiler über ein von Hand drehbares Fixierungsteil (6), das Außen an der Mediumwandung (17, 78) oder durch den Mediumraum (64) geführt ist, festzulegen, um im dritten Schritt über eine Medienzuleitung (12, 14, 18, 22) in einer Reinigungsmaschine mit einem Spül- oder einem Ultraschallverfahren oder außerhalb diese Geräte, Medium über den Mediumraum (64) in verbundenen Adapterkanäle der Korb- und Mediumverteileradapter einzuleiten.

14. Reinigungsverfahren nach Anspruch 14, **dadurch gekennzeichnet, dass** mit Reinigungsvorrichtungen (10, 20, 30, 40, 50, 80, 150) zumindest Hohlraumprodukte (7) ohne Umpacken gereinigt und anschließend sterilisiert werden, wobei ohne Umpacken eine Spülreinigung in Reinigungsautomaten oder Reinigungsgeräten erfolgt und die Verfahren über Vorrichtungen mit festlegbaren und/oder festen Medienverteilern (90, 100, 110, 120, 130, 140) an Medienwänden (60, 70) unterstützt und damit Reinigungsstraßen mit unterschiedlichen Reinigungsverfahren herstellbar sind.

15. Reinigungsverfahren nach Anspruch 14 und 15, **dadurch gekennzeichnet, dass** über zumindest einen Medienanschluss (24, 35) oder U-förmigen Medienanschluss (35) oder über einem Doppelanschlussadapter Reinigungslösung und gepulste Luft oder Dampf in die Reinigungsvorrichtung eingebracht und durchmischt wird, um hiermit zumindest angeschlossene oder eingebrachte medizinische Produkte (7) zu reinigen.

## Claims

1. Cleaning device for hollow instrument cleaning for medical product cleaning and for the operating theatre, comprising a screen basket with apertures and basket adapter (3) and a medium distributor with medium distributor adapter (4, 34) and medium space (64), whereby the basket adapters and the medium distributor adapters are matched to one another, **characterised in that** the cleaning device at least has a fixing part (6), whereby the fixing part (6) comprises a manually rotatable support axle (28), pin, rod or bolt and this being mounted on the outside of a medium wall (17, 78) of the medium distributor or is guided through and held by the wall (17, 78) of the medium space (64) of the medium distributor so that a pressing force of the fixing part (6) with the medium distributor with a screen basket wall (2), a screen basket base (59) or a support surface (1) fastened to one of these can be at least manually controlled, and thereby a coupling of couplable basket adapters (3) with medium distribution adapters (4, 34) is effected in such a way that adapter channels (52, 62) are coupled to one another.

2. Cleaning device according to claim 1, **characterised in that** at least the apertures (41) or the aperture grating (11) of the cleaning device define(s) or co-determine(s) a defined adapter arrangement and holding arrangement and its orientation and provide(s) an aperture size in relation to the channel diameter to be cleaned so that a reliable channel cleaning can take place and whereby the cleaning device is provided with holding parts so that if apertures (41) deviate from the aperture grid (11) or enlarged apertures are present in the device, these apertures are bridged with retaining components (1) in order to fix in place the basket adapters (24, 45, 46, 48, 49, 51) and retaining components (1, 44) or in order to enable adapters (4, 34) of the medium distributor to be introduced into the device from the outside via openings (67).

3. Cleaning device according to claims 1 to 2, **characterised in that** the screen basket of the device contains a surgical set of medical instruments for a medical procedure for use in the operating theatre and the medium distributor consists of at least one manually movable and independently usable medium distributor (90, 100, 110, 120, 130, 140) and this medium distributor is supplied with medium in a cleaning machine or in an ultrasonic bath or outside these devices.

4. Cleaning device according to one of the previous claims, **characterised in that** the screen baskets (10, 20, 150) with basket adapter (24, 45, 46, 48, 49, 51) and the medium distributors (90, 100, 110, 120, 130, 140) with medium distributor adapter (4, 34) are independent cleaning devices and the medium distributors are at least supplied with cleaning medium via fixed supply lines (18, 22) or via flexible supply lines (12).

5. Cleaning device according to one of the previous claims, comprising a fixing adapter, wherein the holding thread (73) has at least a simple thread or multi-start thread for the fixing adapter (44) with an aperture-related shape (68) after the thread, in order to prevent the rotation of the retaining component or the basket adapters in the device and if the retaining component (1) is a strip or a rail and the basket adapters (24, 45, 46, 48, 49, 51) fixed by means of a support bracket (9) as a rotatable retaining component (1) and a medium distributor can be fixed thereto.

6. Cleaning device according to one of the previous claims, **characterised in that** a coupling of different basket adapters and medium distribution adapters (3, 4) takes place and thereby the support axle, the support shaft or the support pin (28) has an anterior thread or a base part (33) with at least the fixing part (6) having sealing elements (27) and/or a retaining groove for springrings (69) and thereby enabling its being guided through a block (29) or a retaining component (66, 76).

7. Cleaning device according to one of the previous claims, **characterised in that** the basket adapters and medium distribution adapters (3, 4) are arranged in series, flat and geometrically, and the plug-in parts (4) have seals (15) or are self-sealing medium distribution adapters (34) in order to seal adapter openings (52) and thereby the plug-in adapters (4) and/or the self-sealing medium distribution adapters (34) support the coupling.

8. Cleaning device according to one of the previous claims, **characterised in that** the medium space (64) is implemented at least in the form of a pipe (5), a cylinder (16) or an angular medium space (5, 38, 60, 70) with at least one flexible medium inlet (12) or a fixed medium inlet (14, 18, 22) or can be additionally implemented as a closable unit via a cock (63), wherein different media can additionally be introduced and mixed via a U-shaped adapter (35) and this latter additionally contains a filter (16).

9. Cleaning device according to one of the previous claims, **characterised in that** medical products (7) are connected to or held in the screen basket or on a support surface (1) of the cleaning device implemented as an adapter strip or a holding plate in the screen basket and thereby at least supporting storage stabilisation and additionally sorting by means of identification markings as well as the management of instruments and connections in the screen basket, whereby additionally electronic sensors or RFID systems are present on at least the basket adapter.

10. Cleaning device according to one of the preceding claims, **characterised in that** the basket adapters contained therein are screwed directly to a holding plate (1) or to an anteriorly sealable nut (54) on a screen basket wall (2) or on a screen basket bottom (59) and can be rotatably arranged over the bottom in order to arrange them individually and prevent an unused escape of media via a closed basket adapter (49) at locations of unused openings.

11. Cleaning device according to one of the preceding claims, **characterised in that** it comprises multi-part basket adapters or screw-in basket adapters, which have a straight or conical channel (52) and a barely protruding sealable channel opening at the front and possesses a connecting part at the rear executed as a hose adapter (46), female Luer lock (51), male Luer lock adapter (48), plug-in adapter (4), cap adapter (45), distribution lances and nozzles (43), closure adapter (49), sleeve adapter (42) and/or fixing adapter (44).

12. Cleaning device according to one of the preceding claims, **characterised in that** at least the fixing of the basket adapters and their adapter arrangement (3, 4) to openings of a wall (2), to a bottom (59) of the screen basket via a groove (37) and a pressing element (27) and/or a shaft ring is supported by a reset force of an elastic pressing element (27) and by a square adapter attachment.

13. Cleaning method, for mechanical medical product cleaning and for the operating theatre, with a cleaning device according to one of the above claims, **characterised in that** in the first step medical products in the sieve basket are connected to the basket adapters or via hoses to the basket adapters, in order to, in the second step, distribute the medium manually via a manually rotatable fixing part (6), the outside being guided to the medium wall (17, 78) or through the medium space (64) in order to, in the third step, introduce medium via a medium supply line (12, 14, 18, 22) in a cleaning machine with a rinsing method or an ultrasonic method or outside these devices via the medium space (64) into connected adapter channels of the basket and medium distribution adapters.

14. Cleaning method according to claim 14, **characterised in that** at least hollow products (7) are cleaned and subsequently sterilised with cleaning devices (10, 20, 30, 40, 50, 80, 150) without repackaging, wherein rinse-cleaning is carried out in automatic cleaning machines or cleaning devices without repackaging and the methods are supported by means of devices with fixable and/or permanently fixed media distributors (90, 100, 110, 120, 130, 140) on media walls (60, 70) and thus cleaning lines with different cleaning methods can be produced.

15. Cleaning method according to claims 14 and 15, **characterised in that** cleaning solution and pulsed air or steam are introduced into the cleaning device via at least one media connection (24, 35) or U-shaped media connection (35) or via a double connection adapter and mixed thoroughly in order to clean at least the connected medical products or medical products introduced therein (7).

## Revendications

1. Dispositif de nettoyage pour le nettoyage d'instruments à cavités pour le nettoyage de produits médicaux et pour la salle d'opération, présentant un tamis comprenant des perforations et adaptateur de tamis (3), et un distributeur de milieu comprenant un adaptateur de distributeur de milieu (4, 34) et une chambre de milieu (64), l'adaptateur de tamis et l'adaptateur de distributeur de milieu étant adaptés l'un à l'autre, **caractérisé en ce qu'**au moins le dispositif de nettoyage présente une pièce de fixation (6), la pièce de fixation (6) étant un arbre (28), une cheville, une tige ou une vis de retenue pouvant être tournée manuellement et celle-ci étant guidée à l'extérieur sur une périphérie de milieu (17, 78) du distributeur de milieu ou guidée et maintenue à travers la paroi (17, 78) de la chambre de milieu (64) du distributeur de milieu, pour que la pièce de fixation (6) puisse au moyen d'une force de pression pouvant être contrôlée manuellement au moins fixer le distributeur de milieu à une paroi du tamis (2), un fond du tamis (59) ou puisse être fixée à une de ces surfaces de support (1) fixées et un couplage de l'adaptateur de tamis (3) pouvant être couplé avec l'adaptateur de distributeur de milieu (4, 34) étant effectué de telle façon que des canaux d'adaptateur (52, 62) sont couplés les uns aux autres.

2. Dispositif de nettoyage selon la revendication 1, **caractérisé en ce qu'**au moins les perforations (41) ou la grille de perforations (11) du dispositif de nettoyage prédéfini ou contribue à déterminer un agencement d'adaptateurs et de supports et leur orientation et fourni, dans son rapport avec le diamètre de canal à nettoyer, une taille de perforation, pour qu'un nettoyage de canal sûr puisse être effectué, et dans lequel le dispositif de nettoyage est muni de parties de support, de telle sorte que, lorsque des perforations (41) divergentes ou supérieures à celles de la grille de perforations (11) sont présentes dans le dispositif, celles-ci sont pontées par des parties de support (1), pour définir les adaptateurs de tamis (24, 45, 46, 48, 49, 51) et les parties de support (1, 44) ou pour que des adaptateurs (4, 34) du distributeur de milieu puissent être introduits de l'extérieur dans le dispositif à travers des perforations (67).

3. Dispositif de nettoyage selon la revendication 1 à 2, **caractérisé en ce que** le tamis du dispositif contient un ensemble chirurgical d'instruments médicaux pour l'utilisation dans la salle d'opération et le distributeur de milieu est au moins un distributeur de milieu (90, 100, 110, 120, 130, 140) pouvant être déplacé manuellement et utilisable de manière autonome et qui est alimenté en milieu dans une machine de nettoyage ou dans un bain ultrasonique ou en dehors de ces appareils.

4. Dispositif de nettoyage selon l'une quelconque des revendications précédentes, **caractérisé en ce que** les tamis (10, 20, 150) comprenant des adaptateurs de tamis (24, 45, 46, 48, 49, 51) et les distributeurs de milieu (90, 100, 110, 120, 130, 140) comprenant des adaptateurs de distributeurs de milieu (4, 34) sont des dispositifs de nettoyages autonomes et les distributeurs de milieu sont alimentés en milieu au moins à travers des conduites fixes (18, 22) ou à travers une conduite flexible (12).

5. Dispositif de nettoyage selon l'une quelconque des revendications précédentes, présentant un adaptateur de fixation, le filetage de retenue (73) d'au moins l'adaptateur de fixation (44) présentant un filetage simple ou multiple comprenant une formation (68) relative à la perforation à la suite du filetage, pour empêcher la torsion de la partie de support ou de l'adaptateur de tamis dans le dispositif, et la partie de support (1) étant une bande ou un rail et les adaptateurs de tamis (24, 45, 46, 48, 49, 51) étant fixés à travers une équerre de fixation (9) comme partie de support (1) rotative et un distributeur de milieu pouvant y être fixé.

6. Dispositif de nettoyage selon l'une quelconque des revendications précédentes, **caractérisé en ce qu'**un couplage de différents adaptateurs de tamis et adaptateurs de distributeurs de milieu (3, 4) est effectué, et l'arbre de retenue, la tige de retenue ou la cheville de retenue (28) présente un filetage avant ou un embout (33) présente au moins la pièce de fixation (6) comprenant des éléments d'étanchéité (27) et/ou une rainure de retenue pour un anneau de retenue (69) et peut être guidé à travers un bloc (29) ou une partie de support (66, 76).

7. Dispositif de nettoyage selon l'une quelconque des revendications précédentes, **caractérisé en ce que** les adaptateurs de tamis et les adaptateurs e distributeurs de milieu (3, 4) sont disposés en série, en surface et géométriquement et les pièces enfichables (4) présentent des joints d'étanchéité (15) ou sont des adaptateurs de distributeurs de milieu (34) auto-étanchéifiants, pour étanchéifier des ouvertures d'adaptateurs (52) et les adaptateurs enfichables (4) et/ou les adaptateurs de distributeurs de milieu (34) auto-étanchéifiants soutenant le couplage.

8. Dispositif de nettoyage selon l'une quelconque des revendications précédentes, **caractérisé en ce que** la chambre de milieu (64) présente au moins un agencement d'un tube (5), d'un cylindre (16) ou d'une chambre de milieu rectangulaire (5, 38, 60, 70) comprenant au moins une arrivée de milieu flexible (12) ou une arrivée de milieu fixe (14, 18, 22) ou peut additionnellement être fermée au moyen d'un robinet (63), additionnellement différents milieux pouvant être introduits ou mélangés au moyen d'un adaptateur en forme d'U (35) et celui-ci contenant additionnellement un filtre (16).

9. Dispositif de nettoyage selon l'une quelconque des revendications précédentes, **caractérisé en ce que** des produits médicaux (7) sont raccordés ou maintenus dans le tamis ou sur une surface de support (1) du dispositif de nettoyage par arrangement au moyen d'une baguette adaptatrice ou d'une plaque de support dans le tamis et ainsi au moins une stabilisation de montage et additionnellement un tri au moyen de marquage et la gestion des instruments et raccords dans le tamis sont soutenus, des capteurs électroniques ou des systèmes RFID étant additionnellement présents au moins sur l'adaptateur de tamis.

10. Dispositif de nettoyage selon l'une quelconque des revendications précédentes, **caractérisé en ce que** les adaptateurs de tamis contenus sont vissés directement avec une plaque de support (1) ou avec un écrou (54) avant pouvant être étanchéifié sur une paroi de tamis (2) ou sur un fond de tamis (59) et peuvent être disposés au-dessus du fond de manière à pouvoir tourner, pour attribuer ceux-ci de manière individuelle et pour empêcher une sortie de milieu non utilisée à des emplacements de perforations non utilisées au moyen d'un adaptateur de tamis (49) fermé.

11. Dispositif de nettoyage selon l'une quelconque des revendications précédentes, **caractérisé en ce que** celui-ci présente des adaptateurs de tamis multi-composants ou des adaptateurs de tamis vissables, qui présentent sur la face avant un canal (52) droit ou conique et une ouverture de canal faisant à peine saillie pouvant être étanchéifiée et possèdent sur la face arrière comme pièce de raccordement un adaptateur de tuyau (46), un adaptateur Luer-Lock femelle (51), un adaptateur Luer-Lock mâle (48), un adaptateur enfichable (4), un adaptateur de bouchon (45), des ajutages et buses de distribution (43), un adaptateur de fermeture (49), un adaptateur de manchon (42) et/ou adaptateur de fixation (44).

12. Dispositif de nettoyage selon l'une quelconque des revendications précédentes, **caractérisé en ce qu'**au moins la fixation des adaptateurs de tamis et de leur agencement d'adaptateurs (3, 4) sur des perforations d'une paroi (2), sur un fond (59) du tamis au moyen d'une rainure (37) et d'un élément de pression (27) et/ou d'une bague d'étanchéité est soutenue par une force de rappel d'un élément de pression (27) élastique et par un embout d'adaptateur carré.

13. Procédé de nettoyage, pour le nettoyage de produits médicaux et pour la salle d'opération, comprenant un dispositif de nettoyage selon l'une quelconque des revendications précédentes, **caractérisé en ce que**, dans la première étape, des produits médicaux sont raccordés dans le tamis aux adaptateurs de tamis ou à travers des tuyaux aux adaptateurs de tamis, pour, dans la deuxième étape, fixer manuellement le distributeur de milieu à travers une pièce de fixation (6) pouvant être tournée manuellement, laquelle est guidée de l'extérieur sur la périphérie de milieu (17, 78) ou à travers la chambre de milieu (64), pour, dans la troisième étape, introduire, à travers une conduite de milieu (12, 14, 18, 22) dans une machine de nettoyage comprenant un procédé de rinçage ou un procédé ultrasonique ou en dehors de ces appareils, un milieu à travers la chambre de milieu (64) dans les canaux d'adaptateurs des adaptateurs de tamis et de distributeurs de milieu.

14. Procédé de nettoyage selon la revendication 14, **caractérisé en ce que**, au moyen de dispositifs de nettoyage (10, 20, 30, 40, 50, 80, 150), au moins des produits à cavités (7) peuvent être nettoyés sans reconditionnement et ensuite stérilisés, un rinçage étant effectué sans reconditionnement dans des laveurs-désinfecteurs ou des appareils de nettoyage et les procédés étant soutenus par des dispositifs comprenant des distributeurs de milieu (90, 100, 110, 120, 130, 140) étant et/ou pouvant été fixés sur des parois de milieu (60, 70) et ainsi des chaînes de nettoyage comprenant différents procédés de nettoyage pouvant être fabriquées.

15. Procédé de nettoyage selon la revendication 14 et 15, **caractérisé en ce qu'**à travers au moins un raccord de milieu (24, 35) ou un raccord de milieu en forme d'U (35) ou à travers un adaptateur de raccord double, une solution de nettoyage et de l'air ou de la vapeur pulsé sont introduits et mélangés dans le dispositif de nettoyage, pour nettoyer avec ceux-ci au moins des produits médicaux (7) raccordés ou introduits.
